# EUROPEAN PATENT APPLICATION

(11) **EP 1 353 182 A2**
(43) Date of publication of application: **15.10.2003**
(21) Application number: 03076051.6
(22) Date of filing: 10.04.2003
(51) Int. Cl.: G01N 33/74, G01N 33/68

(54) **Method of predicting cell-based assay results using binding profiles**

(30) Priority: 12.04.2002 US 372466 P
(71) Applicant: SMITHKLINE BEECHAM CORPORATION, Philadelphia Pennsylvania 19101 (US)
(72) Inventor: Iannone, Marie A., GlaxoSmithKline, North Carolina 27709 (US); Pearce, Kenneth Hugh, Jr., GlaxoSmithKline, North Carolina 27709 (US); Shearin, Jean, GlaxoSmithKline, North Carolina 27709 (US); Simmons, Catherine Ann, GlaxoSmithKline, North Carolina 27709 (US); Svoboda, Daniel Lee, GlaxoSmithKline, North Carolina 27709 (US); Vanderwall, Dana Edward, GlaxoSmithKline, North Carolina 27709 (US)
(74) Representative: Giddings, Peter John, Dr.

(57) **Abstract**

A rapid method of characterizing interactions between compounds and biological molecules in vitro, in order to predict the in vivo characteristics of the compounds, is described. Preferably, the methods utilize labeled microspheres in multiplexed assays.

## Description

### Field of the Invention

The present invention relates generally to a rapid method of characterizing interactions between compounds and biological molecules in vitro, in order to predict the in vivo characteristics of the compounds.

### Background

It is known that biological molecules may take a variety of conformations, and that the binding of a ligand to a biomolecule may stabilize the biomolecule in a particular conformation. For example, The estrogen receptor alpha (ERα) conformation stabilized by estradiol is different than the conformation stabilized by tamoxifen. The conformation of a ligand-bound biomolecule may affect the subsequent biochemical interactions of the biomolecule, including cofactor/accessory protein recruitment, homo- or hetero-dimerization, gene regulation, control of biological processes and cellular/tissue outcomes.

A ligand-bound biomolecule may, for example, interact with a cofactor protein in a different manner, compared to the interaction which would occur between that cofactor protein and the unliganded biomolecule. The variation in cofactor protein binding that occurs due to the conformation of the biomolecule/ligand can be measured using techniques as are known in the art; one such method is to measure and compare the absolute or relative amount of peptide that binds to the various biomolecule/ligand pairs (and/or to the unliganded biomolecule).

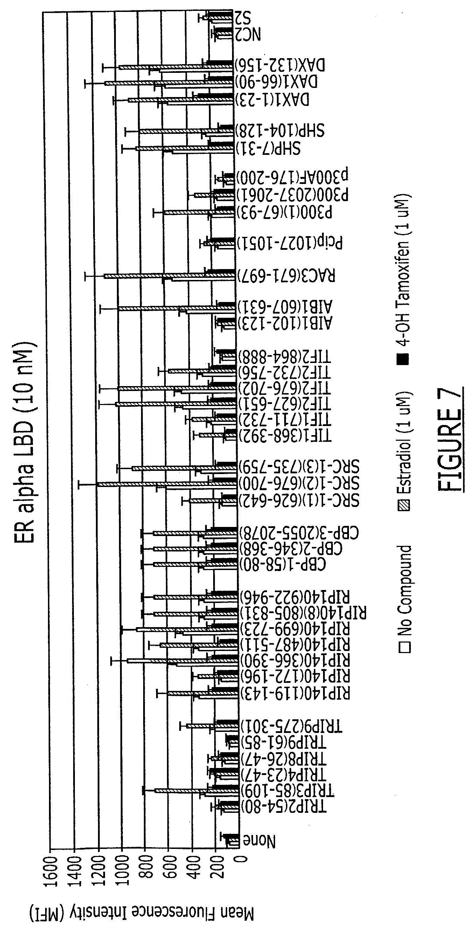

For example, the ERα ligand binding domain (LBD) in an unliganded form will bind to RAC3(671-697) (**see Figure 7**). The percentage of available RAC3 that binds to ERα LBD decreases when the ERα LBD is bound to 4-OH tamoxifen (relative to binding of RAC3 to unliganded ERα LBD) and increases when the ERα is bound to estradiol. The conformation of ERα LBD stabilized by estradiol results in an increase in the amount of RAC3 binding, whereas the conformational change stabilized by 4-OH tamoxifen decreases RAC3 binding (relative to binding in the absence of either tamoxifen or estradiol).

For many biomolecules (including but not limited to nuclear receptors, GPCRs, 7TMs, and ion channels), cell-based assays are known that are reliable indicators of the ability of a compound to activate a certain biomolecule (agonist ), or block activation of the biomolecule (antagonist compound). For example, the MCF-7 breast cell proliferation assay is known in the art for use as a screening assay to identify compounds with ERα agonist or antagonist activity. Thus, the outcome of certain cell-based assays are known to be responsive to modulation (activation or inhibition) of a given biomolecule that is within the cell (either naturally or recombinantly).

In drug discovery research, it is typical to screen vast numbers of chemical compounds using cell-based assays, to identify those compounds with pharmaceutically desirable properties. For example, multiple compounds are known to activate the estrogen receptor activity *in vitro*. Nonetheless, *in vivo* these compounds may have different effects among different tissues types, some of which are pharmaceutically desirable and some of which are not. See, e.g., Shang and Brown, *Science* 295:2465 (2002). For example, it is undesirable for a compound to have proliferative activity in reproductive tissues such as breast or uterus, as one outcome may be an increased risk of cancer. Some compounds, such as tamoxifen, are known to have agonist activity in uterine endometrial cells but act as antagonists in breast cancer cells. It is also recognized that compounds not having activities in reproductive tissues can have other desirable physiological consequences, such as bone protective effects and cardiovascular benefits. Typically, compounds identified as having increased or decreased binding activity *in vitro* must undergo additional cell-based assay screening to identify those compounds most suitable for pharmaceutical development.

As cell-based assays are labor and resource intensive, methods of predicting the cell-based effects of a compound, based on *in vitro* binding assays, are desirable. By identifying those compounds most likely to have desirable cell-based effects, a large series of compounds may be prioritized for subsequent *in vivo* assays, including *in cellulo* (cell and tissue-based assays), and in animal models.

### SUMMARY OF THE INVENTION

A first aspect of the present invention is a method of predicting the effect of a pharmaceutical compound in a cell-based assay that is responsive to modulation of a nuclear receptor. A plurality of reference compounds are selected whose effects in a cell-based assay are known, where the cell-based assay is responsive to modulation of a nuclear receptor. A plurality of binding partners known to bind to the nuclear receptor is also selected, and the binding partners and nuclear receptor are contacted in the presence of a reference compound, under conditions that allow binding. The extent of binding between each binding partner and the nuclear receptor is detected, so that the compound's binding profile can be characterized by the relative differences between the binding results for that compound. These steps are repeated for each of the plurality of reference compounds. For at least one test compound (whose effects in the cell-based assay are not known), the extent of binding of the nuclear receptor to each of the binding partners in the presence of the test compound is determined, such that the test compound's binding profile can be characterized by the relative differences between the binding results for that compound. The binding profile of each reference compound is then compared to the binding profile of the test compound, to identify the reference compound having the binding profile that is most similar to the test compound binding profile. Identification of a reference compound binding profile that is similar to the test compound binding profile indicates that the test compound will achieve results in the cell-based assays similar to the results achieved by the reference compound.

A further aspect of the present invention is a method of predicting the effect of a pharmaceutical compound in a cell-based assay that is responsive to modulation of a G-protein coupled receptor (GPCR). A plurality of reference compounds are selected whose effects in a cell-based assay are known, where the cell-based assay is responsive to modulation of a GPCR. A plurality of binding partners known to bind to the GPCR is also selected, and the binding partners and GPCR are contacted in the presence of a reference compound, under conditions that allow binding. The extent of binding between each binding partner and the GPCR is detected, so that the compound's binding profile can be characterized by the relative differences between the binding results for that compound. These steps are repeated for each of the plurality of reference compounds. For at least one test compound (whose effects in the cell-based assay are not known), the extent of binding of the GPCR to each of the binding partners in the presence of the test compound is determined, such that the test compound's binding profile can be characterized by the relative differences between the binding results for that compound. The binding profile of each reference compound is then compared to the binding profile of the test compound, to identify the reference compound having the binding profile that is most similar to the test compound binding profile. Identification of a reference compound binding profile that is similar to the test compound binding profile indicates that the test compound will achieve results in the cell-based assays similar to the results achieved by the reference compound.

A further aspect of the present invention is a method of predicting the effect of a pharmaceutical compound in a cell-based assay that is responsive to modulation of a 7-transmembrane receptor (7TM receptor). A plurality of reference compounds are selected whose effects in a cell-based assay are known, where the cell-based assay is responsive to modulation of a 7TM receptor. A plurality of binding partners known to bind to the 7TM receptor is also selected, and the binding partners and 7 TM receptor are contacted in the presence of a reference compound, under conditions that allow binding. The extent of binding between each binding partner and the 7TM receptor is detected, so that the compound's binding profile can be characterized by the relative differences between the binding results for that compound. These steps are repeated for each of the plurality of reference compounds. For at least one test compound (whose effects in the cell-based assay are not known), the extent of binding of the 7TM receptor to each of the binding partners in the presence of the test compound is determined, such that the test compound's binding profile can be characterized by the relative differences between the binding results for that compound. The binding profile of each reference compound is then compared to the binding profile of the test compound, to identify the reference compound having the binding profile that is most similar to the test compound binding profile. Identification of a reference compound binding profile that is similar to the test compound binding profile indicates that the test compound will achieve results in the cell-based assays similar to the results achieved by the reference compound

A further aspect of the present invention is a method of predicting the effect of a pharmaceutical compound in a cell-based assay that is responsive to modulation of an ion channel. A plurality of reference compounds are selected whose effects in a cell-based assay are known, where the cell-based assay is responsive to modulation of an ion channel. A plurality of binding partners known to bind to the ion channel is also selected, and the binding partners and an ion channel are contacted in the presence of a reference compound, under conditions that allow binding. The extent of binding between each binding partner and the ion channel is detected, so that the compound's binding profile can be characterized by the relative differences between the binding results for that compound. These steps are repeated for each of the plurality of reference compounds. For at least one test compound (whose effects in the cell-based assay are not known), the extent of binding of the ion channel to each of the binding partners in the presence of the test compound is determined, such that the test compound's binding profile can be characterized by the relative differences between the binding results for that compound. The binding profile of each reference compound is then compared to the binding profile of the test compound, to identify the reference compound having the binding profile that is most similar to the test compound binding profile. Identification of a reference compound binding profile that is similar to the test compound binding profile indicates that the test compound will achieve results in the cell-based assays similar to the results achieved by the reference compound

### BRIEF DESCRIPTION OF THE FIGURES

**Figure 1** is a schematic representation of a method to identify, from a large library of peptides, those peptides that display preferential binding to certain conformations of a biomolecule (in the present case the ERα receptor stabilized in a certain conformation due to the presence of a modulating compound such as estradiol or raloxifene).
**Figure 2** illustrates that phage clones could be identified that bind preferentially to ERα that is conformationally stabilized by one compound.
**Figure 3** lists the names of phage display peptides found to bind preferentially to ERα stabilized by a target compound.
**Figure 4** illustrates that, in the examples described herein, binding of the ERα ligand binding domain (LBD) to peptides coupled to microspheres was detected using flow cytometric analysis to detect the acquisition of orange fluorescence.
**Figure 5** represents the multiplexed molecular interactions that can be utilized to create Binding Profiles as described herein.
**Figure 6** represents the use of flow cytometric analysis of multiplexed microspheres to detect binding of ERα LBD to peptides, as described herein.
**Figure 7** is a Binding Profile of ERα and various cofactor peptides. Extent of binding is represented on the vertical axis by Mean Fluorescence Intensity (MFI); cofactor peptides are referenced on the horizontal axis.
**Figure 8** is the Binding Profile of Figure 7, graphed using basal subtraction to show the increase or decrease in ERα binding in the presence of a compound, compared to basal binding (no compound) .
**Figure 9** is a Binding Profile of ERα and various phage-identified binding peptides.
**Figure 10a** compares the binding of ERα LBD to peptide SRC-1(2) in the presence of various compounds (compared to basal binding; vertical axis) and the results obtained in the ERE cell-based assay (horizontal axis).
**Figure 10b** compares the binding of ERα LBD to peptide RIP 140(3) in the presence of various compounds (compared to basal binding; vertical axis) and the results obtained in the ERE cell-based assay (horizontal axis).
**Figure 10c** compares the binding of ERα LBD to peptide AIB(2) in the presence of various compounds (compared to basal binding;vertical axis) and the results obtained in the ERE cell-based assay (horizontal axis).
**Figure 10d** compares the binding of ERα LBD to peptide TIF2(2) in the presence of various compounds (compared to basal binding; vertical axis) and the results obtained in the ERE cell-based assay (horizontal axis).
**Figure 11a** graphs the results of the MCF-7 cell proliferation assay for varying concentrations of estradiol (horizontal axis), wherein proliferation of a human breast cancer cell line is assessed by [3H]-thymidine incorporation (counts per minute (CPM) on vertical axis). MCF-7 potency = (pEC50 or pIC50)(%cpm max or % cpm min).
**Figure 11b** graphs the results of the MCF-7 cell proliferation assay for varying concentrations of raloxifene (horizontal axis), wherein proliferation of a human breast cancer cell line is assessed by [3H]-thymidine incorporation (counts per minute (CPM) on vertical axis). MCF-7 potency = (pEC50 or pIC50)(%cpm max or % cpm min).
**Figure 12a** compares the binding of ERα LBD to peptide SRC-1(2) in the presence of various compounds (compared to basal binding; vertical axis) and the results obtained in the MCF-7 cell-based assay (horizontal axis).
**Figure 12b** compares the binding of ERα LBD to peptide RIP140(3) in the presence of various compounds (compared to basal binding; vertical axis) and the results obtained in the MCF-7 cell-based assay (horizontal axis).
**Figure 12c** compares the binding of ERα LBD to peptide AIB(2) in the presence of various compounds (compared to basal binding; vertical axis) and the results obtained in the MCF-7 cell-based assay (horizontal axis).
**Figure 12d** compares the binding of ERα LBD to peptide TIF2(2) in the presence of various compounds (compared to basal binding; vertical axis) and the results obtained in the MCF-7 cell-based assay (horizontal axis).
**Figure 13** graphs the results of the Ishikawa cell stimulation assay for varying concentrations of raloxifene and estradiol (horizontal axis), wherein stimulation of endometrial cells is measured by stimulation of intrinsic alkaline phosphatase (A405-blank; vertical axis). %Emax = 100*(A405 compound-blank)/(A405 estradiol-blank).
**Figure 14a** compares the binding of ERα LBD to peptide SRC-1(2) in the presence of various compounds (compared to basal binding; vertical axis) and the results obtained in the Ishikawa cell-based assay (horizontal axis).
**Figure 14b** compares the binding of ERα LBD to peptide T1P3 in the presence of various compounds (compared to basal binding; vertical axis) and the results obtained in the Ishikawa cell-based assay (horizontal axis).

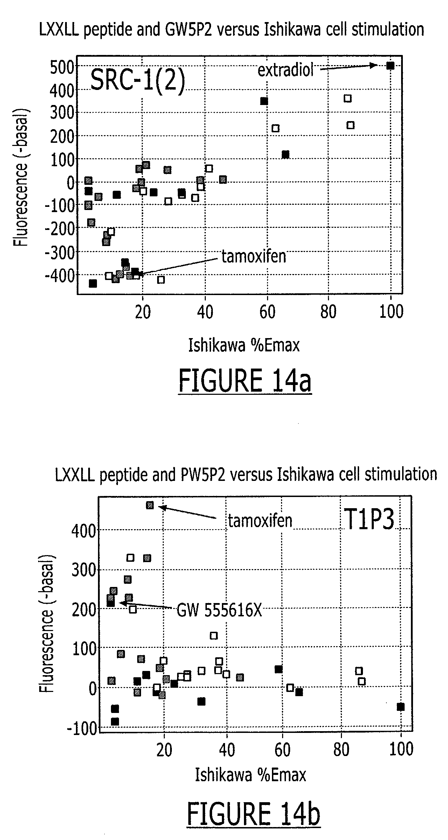

**Figure 14a** compares the binding of ERα LBD to peptide GW5P2 in the presence of various compounds (compared to basal binding; vertical axis) and the results obtained in the Ishikawa cell-based assay (horizontal axis).

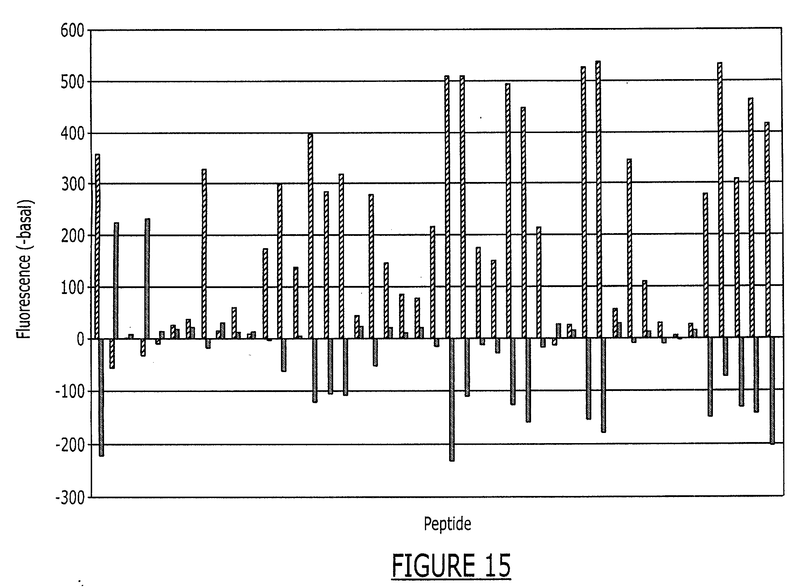

**Figure 15** is a representation of a Binding Landscape consisting of Binding Profiles for two compounds, compared using basal subtraction.

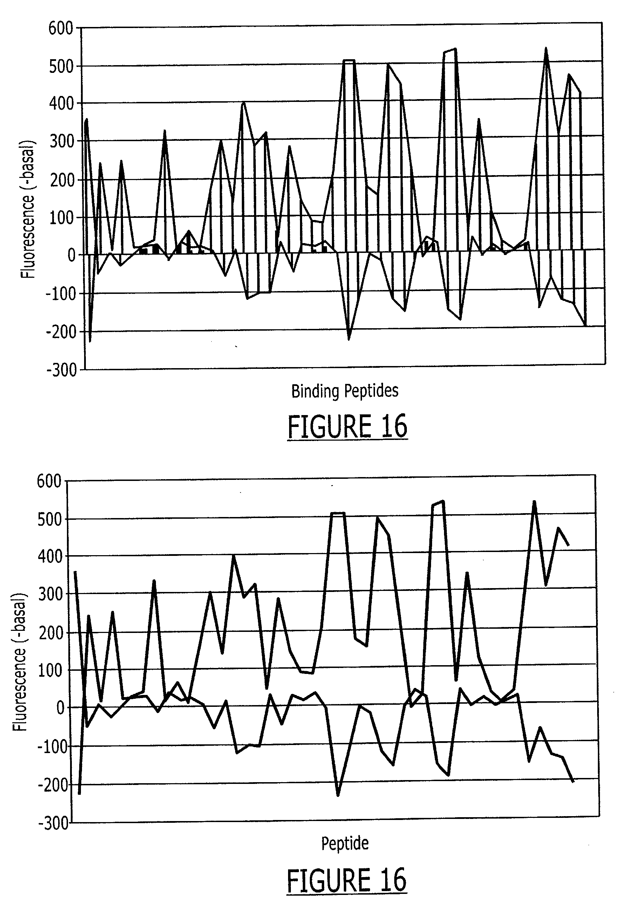

**Figure 16** illustrates how the bar graph of Figure 15 can be converted to a profile plot.

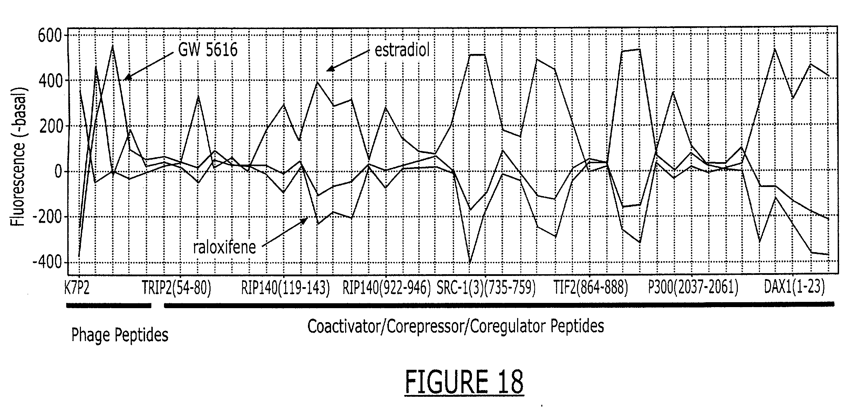

**Figure 17** is the profile plot of the Binding Landscape of Figure 15.
**Figure 18** is a Binding Landscape of three compounds (GW5616, estradiol, and raloxifene).

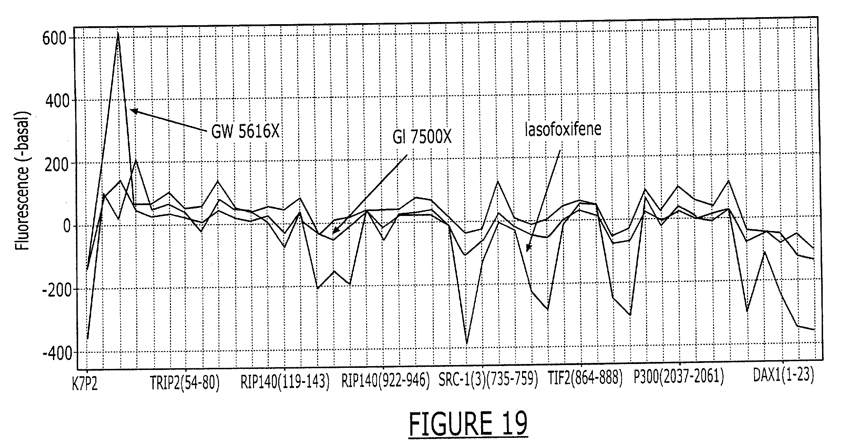

**Figure 19** is a Binding Landscape of three compounds (GW5616, GI7500 and lasofoxifene).

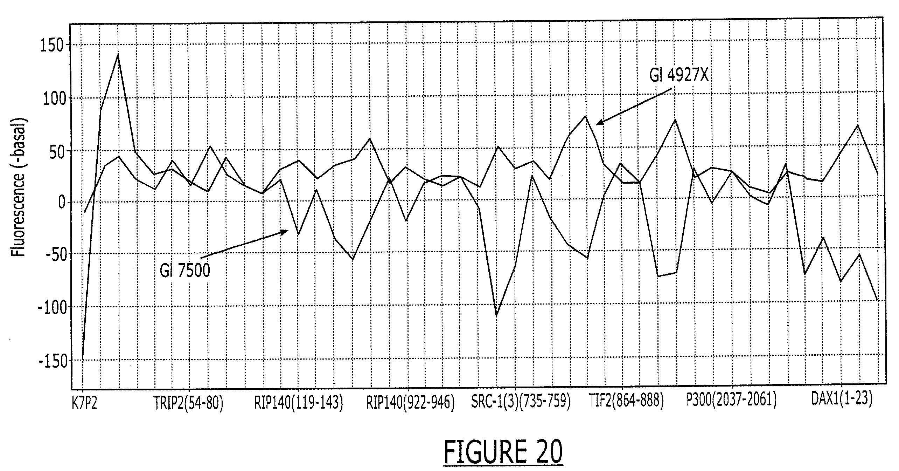

**Figure 20** is a Binding Landscape of two compounds (GI7500 and GI4927). In the Ishikawa cell-based assay, GI7500 = 8.9% and GI4927 = 28.2%.

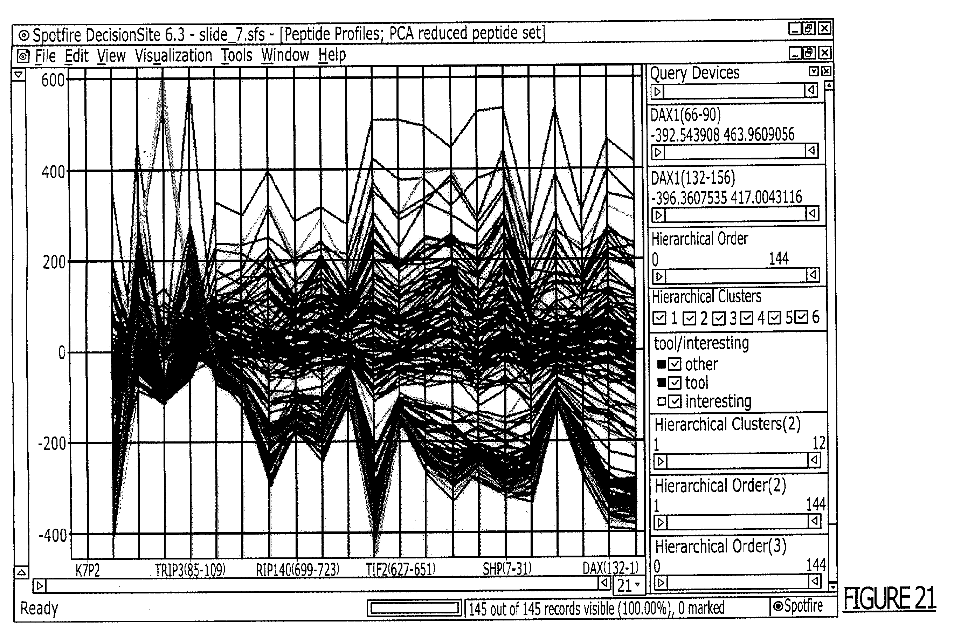

**Figure 21** is a Binding Landscape of 145 compound Binding Profiles.

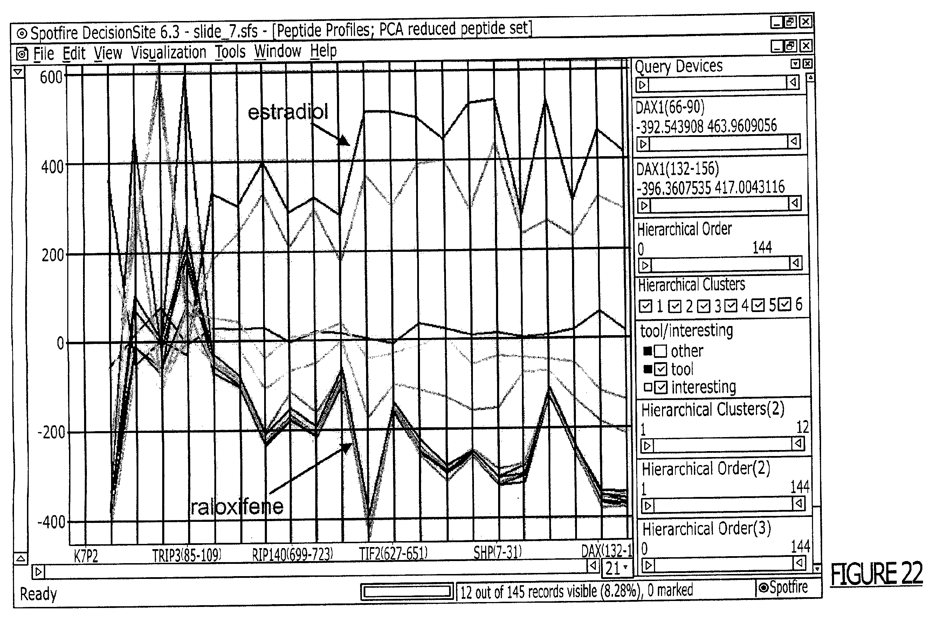

**Figure 22** is a Binding Landscape showing a subset of compounds (reference compounds) of Figure 21.

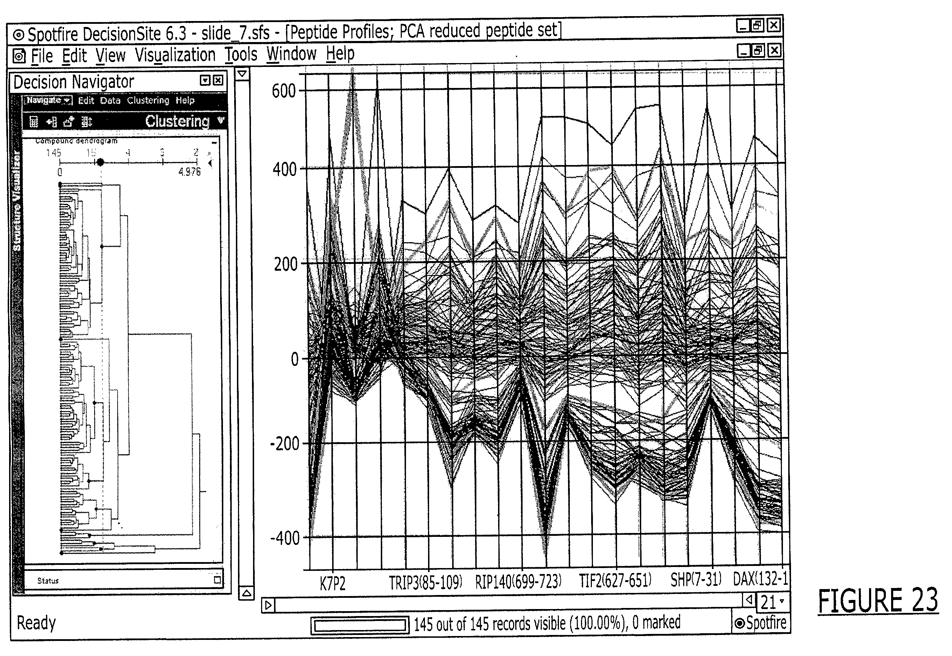

**Figure 23** is a Binding Landscape of 145 compound Binding Profiles, showing the dendrogram utilized in cluster analysis.

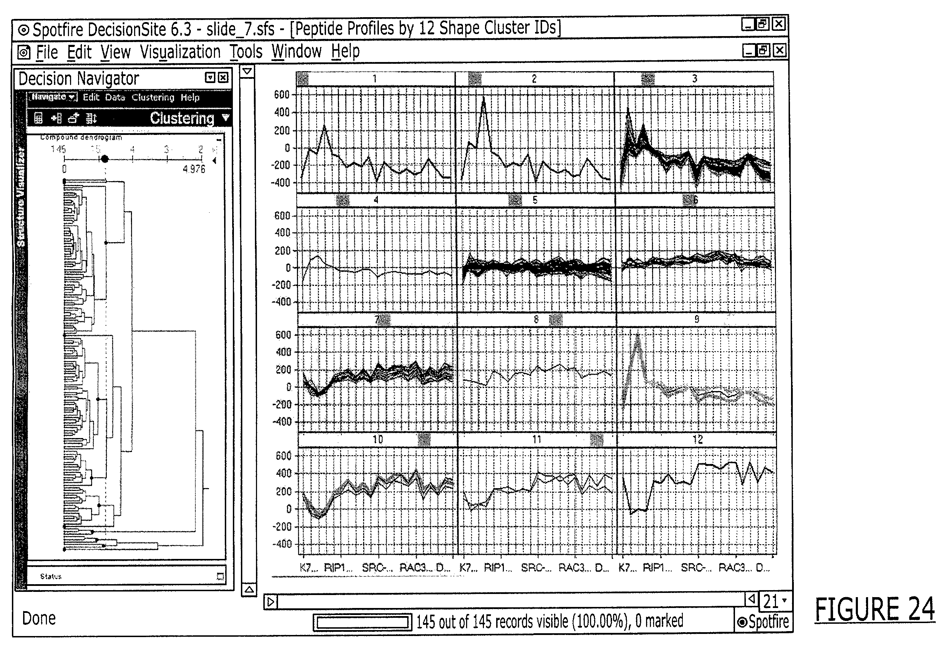

**Figure 24** shows the Binding Landscape of Figure 23 divided into twelve clusters.

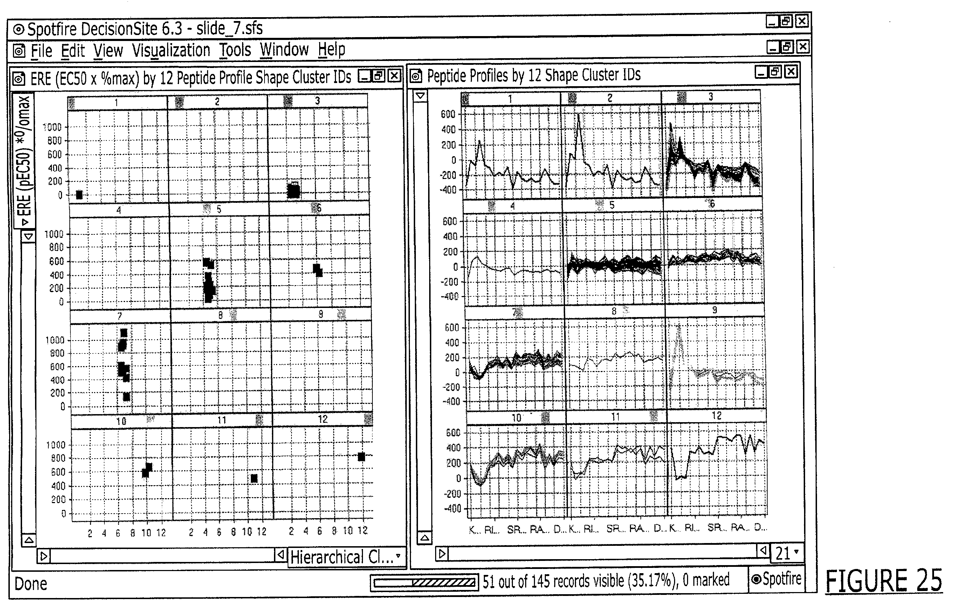

**Figure 25** shows the clusters of Figure 24 correlated to data from the ERE cell-based assay.

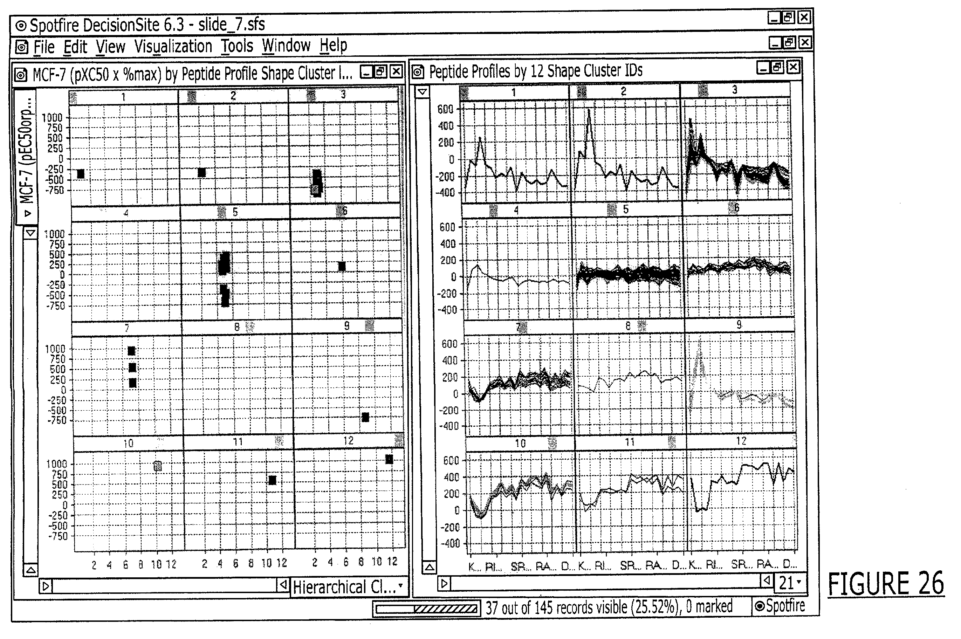

**Figure 26** shows the clusters of Figure 24 correlated to data from MCF-7 cell proliferation assay.

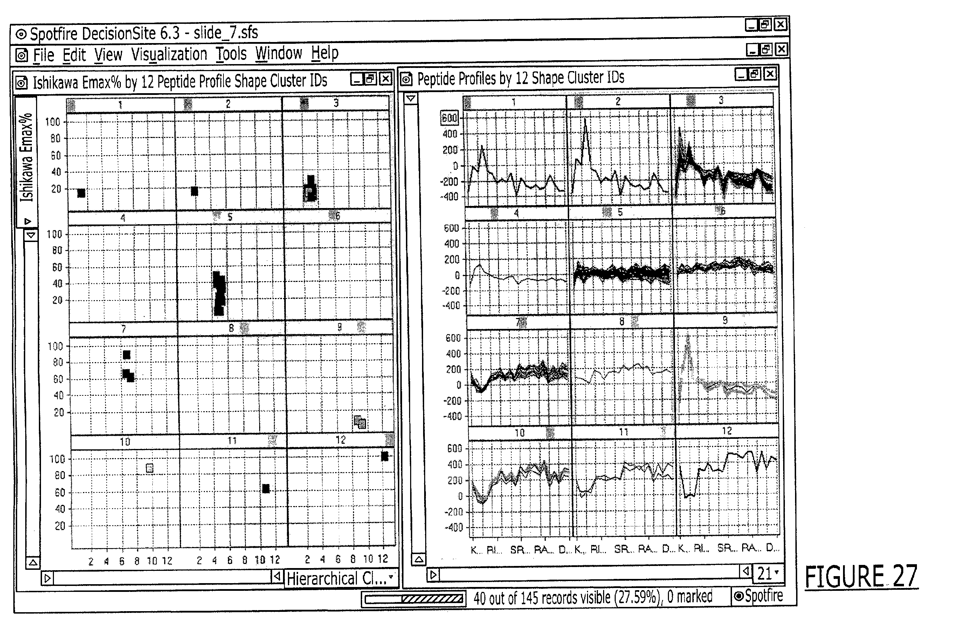

**Figure 27** shows the clusters of Figure 24 correlated to data from the Ishikawa cell-based assay.

### DETAILED DESCRIPTION

Typically, novel or uncharacterized compounds that are believed to bind to, and therefore affect the function of a biological receptor, are studied using cell-based assays, where the cell-based assays have been shown to respond to modulation of that receptor. For example, in studying a compound believed to bind to the ERα ligand binding domain, and affect the function of the estrogen receptor, one skilled in the art would readily know of various cell-based assays in which the outcome of the assay indicates a favorable ERα activity or an unsuitable ERα activity. Due to the time and resources required to conduct cell-based assays, methods of predicting how a compound will act in cell-based assays are useful to prioritize potential pharmaceutical compounds for further study. By selecting compounds most likely to provide the desired cell-based assay results, identification of lead compounds for pharmaceutical development will be facilitated.

The present invention utilizes the conformational plasticity of biomolecules, and the variation in binding of biomolecules to binding partners due to the conformation of the biomolecule, in methods that allow one to predict the results an uncharacterized compound will provide in an associated cell-based assay. In such methods, a selected set of binding partners is assessed to determine the relative binding of each binding partner to a selected biomolecule/ligand pair (a Binding Profile). A plurality of Binding Profiles are created, each Binding Profile utilizing a common set of binding partners and the same biomolecule, but different modulating compounds (ligands) (and optionally a control Binding Profile in which no modulating compound is used). The modulating compounds preferably include at least one known agonist for the biomolecule activity and at least one known antagonist, and more preferably also include modulating compounds that have partial agonist and antagonist activities. At least some of the modulating compounds have been further characterized using cell-based assays (where the cell-based assay is responsive to conformational modulation of the biomolecule). Compounds that have been characterized in at least one such cell-based assay are referred to herein as "reference" or "tool" compounds.

In a Binding Profile, the extent of binding may be assessed as an absolute value, or as a relative value (relative to binding in the control (no binding partner) situation, or as relative binding in the absence of compound (basal peptide binding), or relative to binding to a single reference compound).

The multiple Binding Profiles are then compiled into a Binding Landscape, as described below. The Binding Landscape includes Binding Profiles for both reference (or "tool") compounds, as well as uncharacterized (or "test") compounds. The Binding Landscape then undergoes cluster analysis, to group similar Binding Profiles into clusters.

The present inventors have discovered that, using the above method and comparing the Binding Landscape clusters to associated cell-based results, one can readily predict the results an uncharacterized compound will provide in an associated cell-based assay. Accordingly, the present invention provides methods of analyzing Binding Profiles to detect and predict Structure Activity Relationships (SARs) between modulating compounds and associated cell-based assays.

It will be apparent to one skilled in the art that a Binding Profile may be added to an already completed Binding Landscape (where a common set of binding partners and the same biomolecule are utilized), creating a "new" Binding Landscape which can be subjected to cluster analysis. In other words, adding a Binding Profile to a Binding Landscape does not require that the Binding Profiles for each reference or test compound be recreated. Similarly, where it is desired to add a binding partner to the Binding Landscape analysis, the additional binding partner can be studied with each biomolecule/ligand pair, and this new binding information added to existing Binding Profiles, and used to create a new Binding Landscape. In other words, the data produced and used in a first Binding Landscape analysis may be re-utilized in a second Binding Landscape analysis without the necessity of re-creating the in vitro studies that initially provided the data.

Thus in one embodiment of the present invention, the effect of various modulating compounds (reference and test compounds) on the binding of a biomolecule target to a plurality of binding partners is investigated, and used to predict the effects of the uncharacterized compounds in associated cell-based assays. Suitable biomolecules that may be studied by the present methods include, but are not limited to, nuclear receptors, GPCRs, ion channels, and 7-transmembrane receptors. The biomolecule may be full-length or may be a fragment of the biomolecule that contains a binding domain.

The present method of acquiring conformation-based binding data to predict cellular responses can be applied to various classes of biomolecule receptors where activities such as conformational changes, associations of protein or DNA binding partners, or homo- hetero-dimerization can be modulated by small molecules. Examples of such receptors suitable for use as target biomolecules in the present methods include seven transmembrane receptors, ion channels, transcription factors or repressors, and other signaling molecules.

For example, a typical seven transmembrane G-protein coupled receptor or □-adrenergic receptor can be recombinantly expressed, purified, and then reconstituted into vesicles or liposomes (Levitzki, (1985) *Biochimica et Biophysica Acta*. 822: 127-53; McIntire et al. (2002) *Methods in Enzymology* 343: 372-93). The receptor will be incorporated into the lipid vesicle in one of several orientations, such as extracellular side out and intracellular side in or vice versa. This protein can be labeled with an appropriate fluorophore. Binding partners, such as G-proteins, kinases, transcription factors, or peptides identified by an affinity selection technique such as phage display, can be biotinylated and then attached to a streptavidin-coated fluorescent microsphere. A small molecule that binds to the fluorescently-labeled transmembrane receptor can induce a conformational change in the receptor which enables association or dissociation with a number of binding partners that are attached to a particular fluorescent bead. These binding events are related to stimulation of a signaling cascade and ultimately are related to an effect that a small molecule has on a cell. This approach of obtaining a membrane receptor and using lipid vesicle reconstitution can be applied to other classes of receptors such as ion channels, hematopoetic single membrane-spanning receptors, immunoglobulin-like receptors, ATPases, and/or integrins.

The binding partners used to create a Binding Profile may include full length coactivator, coregulator, or corepressor proteins for the biomolecule, phage display peptides that have been determined to bind to the biomolecule; as well as fragments of coactivator, coregulator, or corepressor proteins that are known to bind to the biomolecule, or that contain known binding motifs for that biomolecule. In addition, proteins and DNA fragments that are discovered to be binding partners or promoter elements, respectively, may also be used to create a Binding Profile.

Suitable modulating compounds include known agonists, antagonists, or compounds having partial agonist and partial antagonist effects on the target biomolecule, and test or uncharacterized compounds whose effects on, or binding to, the target biomolecule are unknown.

The present methods utilize the multiplexed binding assay as described in **WO 01/75443 (Consler et al., Glaxo Group Limited)** to generate a Binding Profile for a preselected receptor and a set of binding peptides, in the presence or absence of a modulating compound. See also Iannone et al., Cytometry 44:326 (2001); Iannone, Clinics in Laboratory Medicine, 21(4): 731 (2001).

A Binding Profile is thus a compilation of binding data for a particular receptor or biomolecule, a plurality of binding partners, and the presence of a particular modulator (or no modulator), or combinations of modulators. Preferably, what is measured is, for each binding partner, the percentage of available binding partner moieties that are boudn to the biomolecule under the assay conditions, in the presence of a modulating compound (or no modulating compound). The individual binding assays are conducted under similar conditions, so that results can be compared within a Binding Profile. For example, using a multiplexed system where a plurality of interactions are studied in a single volume aids in ensuring the use of similar conditions. For ease of interpretation a Binding Profile may be represented graphically, e.g., as a bar graph or profile plot (see **Figure 15**). The Binding Profile allows one to compare the extent of binding, among a plurality of binding partners, to a plurality of ligand-bound biomolecules. See **WO 01/75443**, the contents of which are incorporated herein by reference in their entirety.

According to the present invention, multiple Binding Profiles are compiled into a Binding Landscape. The individual Binding Profiles must contain a common set of binding partners and the same target biomolecule, but differ in the modulating compound used. For Binding Profiles that are compiled into a single Binding Landscape, the binding assays are conducted under similar conditions such that binding results can be compared among the Binding Profiles ("similar conditions" as will be apparent to one skilled in the art).

Once a Binding Landscape has been prepared for a biomolecule, a Binding Profile for a test compound can be assessed using the Binding Landscape, to predict the test compound's activity in associated cell-based assays.

It will be apparent to one skilled in the art that an initial Binding Landscape may be prepared using only reference compounds, and subsequently Binding Profiles for individual test compounds may be added to and analyzed with the Binding Landscape. Alternatively, an initial Binding Landscape may be prepared that includes multiple reference compounds as well as one or more test compounds.

### Binding Profile Example: ERα

A combination of three Binding Profiles uisng the ERα LBD as the biomolecule target is shown in **Figure 7**. Each Binding Profile represents a set of 39 binding peptides (synthesized fragments of biotinylated corepressors, coactivators and coregulators) as well as a 'no-peptide' control. The Binding Profiles were run with either estradiol or 4-OH Tamoxifen as the modulating compound, and a basal or control Binding Profile was run without any modulating compound. The vertical axis shows Mean Fluorescence Intensity (MFI) and is an indicator of the percentage of each peptide class that was bound by ERα LBD.

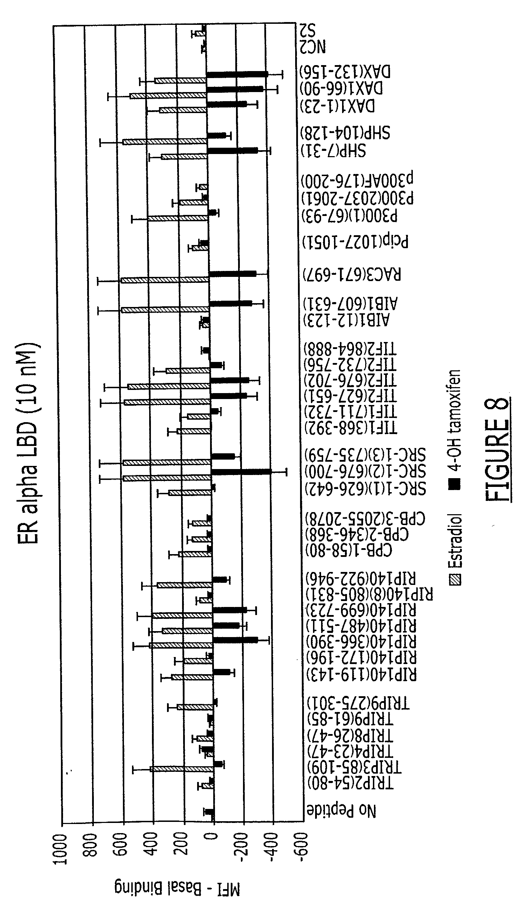

**Figure 8** shows the data as graphed in Figure 7, but graphed to show the change in binding, with the basal (no modulator) binding subtracted (basal subtraction). It is apparent from Figure 8 that the presence of 4-OH tamoxifen decreases or only slightly increases binding of the tested binding partners, compared to basal levels. In contrast, estradiol increases binding of ERα to most of the binding peptides.

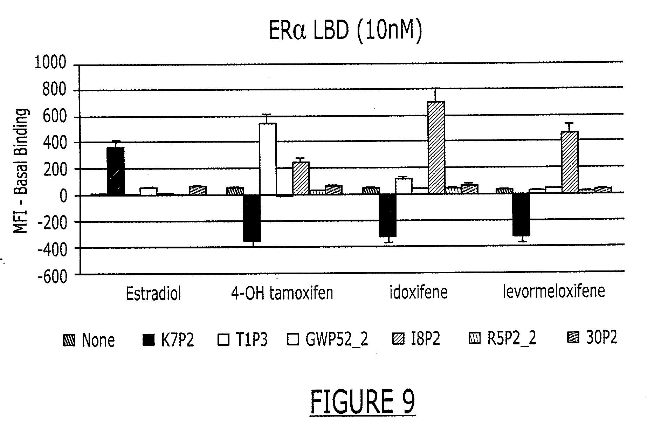

**Figure 9** shows ERα LBD binding to six phage-identified peptides in the presence of four different modulating compounds. K7P2 binding is increased in the presence of estradiol, and not in the presence of the anti-estrogen compounds 4-OH tamoxifen, or in the presence of idoxifene or levormeloxifene. As used herein, a compound with estrogenic effects is one that stabilizes the estrogen receptor in a conformation that promotes binding to coactivators, whereas a compound with anti-estrogenic effects stabilizes the receptor in a conformation that decreases binding to coactivators. Accordingly, K7P2 is identified as a useful peptide to use in Binding Profiles, to indicate compounds that have estradiol-like (estrogenic) effects on ERα.

The present inventors discovered that Binding Profiles created using known receptor modulators (where the effects of those modulators are also known in cell-based assays responsive to modulation of the target biomolecule), could be analyzed to predict the cell-based activity of an uncharacterized modulator. In other words, raloxifene + ERα produces a certain Binding Profile for a selected set of binding proteins. Raloxifene used in cell-based assays will also produce consistent results in a cell-based assay, where that cell-based assay is responsive to ERα ligands. By comparing the ERα Binding Profile of an uncharacterized ERα modulating compound with Binding Profiles of characterized ERα modulating compounds, it is possible to predict how the uncharacterized compound will act in cell-based assays. The prediction is not based on the comparison of any one binding peptide, but is based on a comparison of the collection of responses to the set of binding peptides (the Binding Profile). This method is also useful for rapid determination that an uncharacterized or novel ERα modulating compound is different than a characterized compound, by determining that its Binding Profile is unique compared to that of the characterized compound, or collection of known compounds.

While not wishing to be held to a single theory, the present inventors believe that the results in cell-based assays are dependent on the relative extent of *in vivo* binding among multiple binding partners, and have determined that *in vitro* binding assays are predictive of cell-based behavior. Further, the present inventors have determined that binding peptides identified by screening phage display libraries (i.e., peptides that may or may not be present in the cell-based assays) also have predictive value in the present methods. The phage display libraries to be screened may be random, unfocused libraries.

Using the high-throughput generation of Binding Profiles to predict the activity of an uncharacterized compound in a cell-based assay allows the selection of the most promising new candidates from a library of candidate compounds. In this manner, high-throughput methods can be used to prioritize candidate compounds for testing in more laborious cell-based assays, and/or to exclude the unpromising or least desirable candidates.

### Binding Landscapes

Using Binding Profiles generated as above, a Binding Landscape was compiled, and was then analyzed to predict the effect of uncharacterized compounds in ERα -responsive cell based assays.

As shown in **Figure 15**, the peptide Binding Profiles for two or more modulator compounds (using the same target biomolecule and set of binding partners), can be represented as a Binding Landscape, where one dimension (height of each bar in Figure 15) represents the increased or decreased amount of peptide binding to the target biomolecule for a particular biomolecule/ligand combination. In **Figure 15**, binding is increased or decreased compared to binding in the absence of any modulating compound (the basal binding). It will be readily apparent to those skilled in the art that the precise graphical representation of a Binding Landscape may vary; **Figure 15** is an example of a Binding Landscape in a bar graph format, with the vertical axis representing increase or decrease in binding over basal binding.

Alternatively, a 'basal' binding level could be determined using a reference modulating compound (rather than no compound); the increase or decrease in binding achieved in the presence of other compounds would be reported as the increase/decrease over that achieved with the reference compound.

As shown in **Figures 16 and 17,** the bar heights of Figure 15 may be made continuous, to provide a better graphical representation of the distinctive Binding Landscape pattern. In **Figures 16 and 17,** the vertical axis represents the amount of binding minus the basal level of binding (as indicated by the fluorescence measurement).

By preparing a Binding Landscape comprising multiple reference or tool compound Binding Profiles, and comprising at least one Binding Profile for an uncharacterized compound, one can predict the uncharacterized compound's effects in associated cell-based assays. In this manner, by analyzing uncharacterized compounds in Binding Landscapes, one can identify novel compounds that are most likely to function in cell-based assays in a desirable manner. Alternatively, one can identify uncharacterized compounds that are likely to provide cell-based assay results that are unlike any of the reference compound results, and to identify those with undesirable cell-based assay effects.

**Figure 18** shows a Binding Landscape for ERα compiled of the Binding Profiles of estradiol, raloxifene, and GW5616 (the set of binding peptides is represented on the horizontal axis). It is readily apparent that the Binding Profile of GW5616 is more similar to raloxifene than to estradiol, yet there are features of the Binding Profile of GW5616 that make it distinct from both tool compounds.

**Figure 19** compares the Binding Profiles for two experimental Selective Estrogen Receptor Modulators (SERMs) and lasofoxifene. As shown, Compound 7500 gives a unique peptide binding profile. When tested in the Ishikawa cell based assay, Compound 7500 gave %Emax = 8.9% ± 2.7% These cell based assay results for Compound 7500 were also distinct from the results obtained with raloxifene.

**Figure 20** compares the Binding Profiles for two SERM compounds, 7500 and 4927. Note that both Figure 19 and Figure 20 show the same binding profile for compound 7500 but using different scales. The overall appearance of the Compound 7500 Binding Profile differs between these Figures only because the scale is different, and illustrates that Binding Profile data may be displayed in different visual formats. Within a Binding Landscape, however, the individual Binding Profiles must be graphically represented in the same manner, and each must contain a common set of binding partners.

### Similarity of Binding Profiles

As used herein, "similar" Binding Profiles are those that are grouped together by a clustering analysis. Clustering, as applied here, is a quantitative approach used to characterize and segregate some number of individual objects into a smaller number of groups, or clusters. Members of a cluster have more in common with one another than with objects in other clusters. One common use or interpretation of a clustering analysis is that members of a cluster will exhibit similar characteristics or behaviors outside of those used as a quantitative measure in the clustering analysis itself.

The criteria by which the objects are compared are the 'descriptors', and in the present invention are the binding profile data. Each set of binding data for one binding partner constitutes one 'dimension' in 'descriptor space'.

This clustering analysis has three components: (1) the quantitative measure of similarity between peptide binding profiles of individual compounds, (2) the quantitative measure of similarity between clusters, and (3) the particular methodology for forming clusters based on the similarity within (1) and between candidate clusters (2).

As an example, consider a matrix of peptide binding data arranged so that the data for each compound lies along one row, where the binding measurements for each peptide occupy a series of adjacent columns. A profile for one compound can be characterized by the relative differences between the different peptide binding results. This can be quantitated as the slope (or gradient)between adjacent peptide binding results. The quantitative measure of similarity between two compounds (1) is the euclidian distance between the slopes of those compounds' profiles between each pair of result columns. THis method focuses on the pattern of relative activity across multiple columns of data, without consideration of the absolute magnitude. This comparison between two profiles is termed the 'shape' based clustering in Spotfire Decision Site, a commercial application useful for such analyses.

To quantitate the similarity, or 'distance' between two clusters (2), the mean similarity between all members of the two clusters is calculated. Finally, a hierarchical agglomerative method can be used to carry out the actual formation of clusters. This approach begins with each individual compound as a cluster, and by way of the similarity measrues described above, combines individuals to form clusters. Iteratively, all members can be combined into one cluster, and a tree-like structure or dendogram can be used to illustrate the relationship between the clusters at each step of combination or 'agglomeration'. The choice of the number of clusters ultimately used to represent the data may be made by selecting a boundary line through the cluster tree. This is subjective, but targets a balance between having many homogeneous profiles within any given cluster, and having too many clusters containing one or few compounds.

### Dimension Reduction in Binding Landscape

The present inventors determined that in some Binding Landscapes data from a subset of the binding partners may provide the majority of the variation in binding among the biomolecule/ligand pairs studied. Where, for example, a peptide bound to every biomolecule/ligand pair in essentially the same manner (or did not bind to any ), that binding partner would not contribute in a meaningful way to predicting cell-based assay results. Additionally, where multiple binding partners mimicked each others' responses to the biomolecule/ligand pairs studied (i.e., were correlated), most of those should be removed from the Landscape analysis to reduce possible bias due to overrepresentation of the binding effects represented by these binding partners.

For example, in a study described herein using ERα LBD, a set of 45 binding peptides (plus a no-peptide control = 46 peptide variables), and 145 different modulator compounds, it was determined that 21 of the original 46 peptide variables accounted for ≥ 99% of the variation detected. Subsequent clustering analysis was conducted with the Binding Profile data for these 21 peptide variables.

Accordingly, it is preferable that dimensional reduction analysis be carried out on a Binding Landscape, to identify and remove correlated data, and to remove the least informative binding partners. Dimensional reduction may be accomplished using any suitable statistical method as is known in the art. One such method is Principal Components Analysis (PCA), which may be accomplished using the JMP program (available from SAS Institute, Cary, NC). Preferably PCA will be conducted and those components will be selected that represent at least about 80% of the variation in the data, more preferably at least about 85%, 90%, 93%, 95% or more, most preferably 98% or 99%. For each of the chosen components, binding partners representing contributions of more than 25% to each of these components are preferably selected as data dimensions in the subsequent analysis. As will be apparent to those skilled in multivariate analysis, methods other than PCA, and/or software other than JMP may be used for dimensional reduction within a Binding Landscape.

As used herein, "binding peptides" are peptides that bind preferentially to at least one form of a biomolecule target. The biomolecule target may be in a liganded or unliganded form, and binding of the binding peptide to the biomolecule may vary depending on the conformation of the biomolecule.

As used herein, a "biomolecule" refers to a molecule that functions within a living organism by interacting with other molecules, including protein/protein interactions, nucleic acid/protein interactions, and nucleic acid/nucleic acid interactions. Furthermore, these functional interactions are frequently modulated by an additional molecule or molecules to form a macromolecular functional unit. Biomolecules include but are not limited to nuclear receptors, GPCRs, 7TMs, and ion channels. Assays that have traditionally been used to evaluate these functional interactions include enzyme activity assays, ligand binding assays, endpoint assays, kinetic binding assays, competitive binding assays, hybridization reactions, BIACORE, homogeneous time-resolved fluorescence, scintillation proximity assays, and others.

As used herein, a cell-based assay "responsive" to modulation of a given biomolecule is one in which the measured outcome of the assay is altered depending on the activity of that biomolecule. In other words, the cell-based assay is useful to indicate when functional modulation of that biomolecule has occurred.

As used herein, a "test" or "uncharacterized" compound is one whose activity in a particular cell-based assay is not known. An uncharacterized compound may have a known chemical structure or an unknown structure. A compound may be "uncharacterized" with respect to one cell-based assay, but characterized with respect to a different cell-based assay.

As used herein, a "reference" or "tool" compound is one whose activity in a particular cell-based assay is known. A characterized compound may have a known chemical structure or an unknown structure. A compound may be "characterized" with respect to one cell-based assay, but uncharacterized with respect to a different cell-based assay.

As used herein, a "modulating compound" for a particular biomolecule is one that binds to that biomolecule and induces a conformational change. For example both estradiol and raloxifen are modulating compounds for ERα.

As used herein, "in a single assay" means a single assay volume (e.g., a single tube or well). The single assay can be a multiplexed assay in which simultaneous, or near simultaneous, determinations of binding events can be measured from the same assay process. For example, numerous first members, second members, and additional members of numerous binding complexes can be added to the same assay process for subsequent identification and analysis.

"Relative binding" means the amount of binding that occurs between one binding pair as compared to another binding pair. For example, one first member of a binding pair may have relatively higher binding to one specific second member than to a different second member. One first member may have higher binding to one second member as compared to the binding between a different first member and a different second member. Similarly, a specific first and a specific second member may have higher relative binding compared to a different first member and different second member. The amount of binding can include the absence of binding.

"Conditions that allow binding" of binding components, (biomolecules, binding partners, modulating agents) refer to those parameters that are conducive to the binding event, as would be understood by one skilled in the art. Such conditions can include, for example, pH, time, temperature, and buffer composition. Such conditions do not require that all binding components are involved in binding interactions, only that the conditions allow for specific binding interactions to occur

By "binding pairs" is meant at least two binding members that have bound together, or are capable of preferentially binding together. A modulating agent may bind to either member of a biomolecule-binding partner binding pair, and may prevent or enhance binding between these members of the binding pair. By "binding complex" is meant at least a binding pair and, optionally, a third member and/or a modulating agent. In some cases the modulating agent may allow binding between two members of a binding pair that do not form a binding pair in the absence of the modulating agent. A biomolecule-ligand pair refers to a biomolecule and a ligand (modulator or compound) capable of binding to the biomolecule. When bound, this may be referred to as a "liganded biomolecule".

As used throughout, the "contacting" step of the present invention is preferably *in vitro*.

Microspheres used in the present methods may include one or more fluorescent "labels mixed together to constitute a specific "label" for a subset of microspheres. For example, it is well known in the art that microspheres can be labeled with two or more fluorochromes mixed together in varying concentrations, such that each specific label has a specific concentration of each fluorochrome. It is the specific concentrations of the various fluorochromes together that provide a spectrum of labels that can be used to distinguish among subsets of labeled microspheres.

Microspheres used in the present inventions preferably have a plurality of the same molecule (binding partner or biomolecule) coupled to it. By "a set of microspheres" is meant a plurality of microspheres consisting of subsets of microspheres labeled with distinguishable labels. By coupling each subset to a specific first member, or a plurality of the same first member, a specific label for each first member species is provided.

"Coupled directly or indirectly" will be understood by one skilled in the art to include various methods for coupling. For example, binding partners or biomolecules used in the present methods can be coupled directly or indirectly to microspheres, using streptavidin, biotinylation or maleimide or can be carboxylated. One skilled in the art would recognize that other coupling agents can be used.. See, e.g., WO 99/19515 and WO 99/37814, which are incorporated herein by reference in their entirety for types of functional groups that can be used for coupling agents to the microspheres. Optionally, a linker can be used between the microsphere and the agent.

Labels can also be coupled to the microspheres, agents to be screened, or second members using a variety of methods known in the art. As used throughout, "label" refers to a moiety (e.g., a hapten) that provides a means for labeling, as well as radiolabels, and fluorescent labels. Thus, labels can be radiolabels, dyes, fluorescent labels, or a combination thereof. One skilled in the art would recognize that numerous fluorochromes are available for use in the present method.. See, e.g., WO 99/19515 and WO 99/37814, which are incorporated herein in their entirety for types of fluorescent dyes and fluorochromes that can be used as labels.

One skilled in the art would recognize that a variety of types of microspheres can be used in the methods of the present invention. See, e.g., WO 99/19515 and WO 99/37814, which are incorporated herein in their entirety for types of microspheres and methods of making and using same. For example, the microspheres can be polystyrene-divinylbenzene microspheres.

By "detecting the presence" of a label means detecting any amount of label. Thus, in some cases the amount of label may be an absence of the label.

As used throughout, "a binding profile" is a systematic summary of binding data such that the binding profile indicates the relative binding between a first member and second members of various binding pairs. The binding of the first and second members may be influenced by the presence of a third agent, such as a modulating compound.

As used herein, binding partners may be selected from a group comprising cofactors, receptors, receptor ligands, proteins, peptides, protein domains, oligonucleotides, transcription factors, nucleic acids, small molecules, and small compounds, any of which can be synthetic, modified, or naturally occurring.

By "small molecules" is meant natural or synthetic organic molecules less than about 1000 daltons and, more preferably, less than about 500 daltons. Small molecules, for example, include estradiol, cyclic nucleotides, retinoic acid, steroid hormones, amino acids, neurotransmitters (e.g., norepinephrine, epinephrine, acetylcholine), and numerous other compounds.

As used herein, "receptor" includes but is not limited to orphan receptors or nuclear receptors. As used herein, "orphan receptors" are molecules identified as having receptor or receptor-like domains or tertiary structures but lacking a known function. Thus, the present methods can be used to characterize the binding profile of an orphan receptor in an effort to characterize the function of the receptor. Nuclear receptors include, for example, all known receptors identified by the Nuclear Receptors Nomenclature Committee, 1999. See Vincent Laudet, Johan Auwerx, Jan-Ake Gustafsson, and Walter Wahli; A Unified Nomenclature System for the Nuclear Receptor Superfamily, Cell (1999) 97: 161-163, which is incorporated herein in its entirety by reference for the identification of known nuclear receptors. Nuclear receptors also include, for example, receptors that have yet to be identified but have at least one function or have at least one characteristic of known nuclear receptors.

"Cofactors" or "coregulators" as used throughout can refer to coactivators, corepressors, or a combination of coactivators and corepressors. Examples of nuclear receptor cofactors include, for example, those identified in Daniel Robyr, Alan P.Wolffe and Walter Wahli, Nuclear Hormone Receptor Coregulators in Action: Diversity for Shared Tasks, Mol.Endocrinol. (2000) 14: 329-347, which is incorporated herein in its entirety for examples of nuclear receptor cofactors.

By "conditions that allow formation of detection products" means conditions in which first members can bind to second members, and preferably conditions in which modulating agents can bind to either the first member, second member, third member, or some combination thereof. Such conditions do not require that all first and second members bind or that all third members bind, only that the conditions allow for specific binding interactions to occur. These conditions include, for example, pH, time, temperature, and buffer composition, which allow binding between the members and agents of interest.

A "set of microspheres" comprises one or more subsets of microspheres wherein each subset comprises a plurality of microspheres labeled with the same label or combination of labels and wherein each label or combination of labels is specific for that subset.

The present invention is more particularly described in the following examples which are intended as illustrative only, as numerous modifications and variations therein will be apparent to those skilled in the art.

Throughout this application, various publications are referenced. The disclosures of these publications in their entireties are hereby incorporated by reference into this application in order to more fully describe the state of the art to which this invention pertains.

Although the present process has been described with reference to specific details of certain embodiments thereof, it is not intended that such details should be regarded as limitations upon the scope of the invention except as and to the extent that they are included in the accompanying claims.

### EXAMPLE 1

### Identification of Binding Peptides using Phage Display ("Affinity Selection")

Phage display was used to identify binding peptides that preferentially bind to certain conformations of the ERα receptor. Such peptides are useful in the production of Binding Profiles for that receptor. Methods of phage display and selection are known in the art (see e.g., US Patent 5,837,500, WO 97/35196; WO 98/19162; Norris et al., *Mol. & Cell. Biol.* 19(12):8226-39, 1999; Wijayaratne et al. *Endocrinology 140(12):5828-40, 1999;* Norris et al., *Science* 285(5428):744-6, 1999*;* Paige *et al, Proc. Natl Acad. Sci. (USA)* 96(7):3999-4004, 1999.

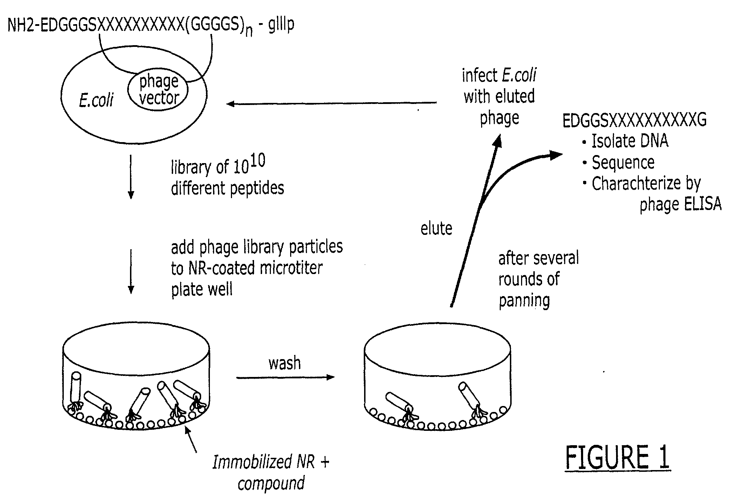

As shown in **Figure 1,** a phage display library of approximately 10¹⁰ different peptides was constructed and screened. The phage library particles were contacted with the target receptor of interest (ERα ligand binding domain) in both the unliganded form and the liganded (receptor + modulating compound) form. In this example, modulating compounds used included estradiol, tamoxifen, raloxifene, and GW5638.

After washing to remove unbound phage and then eluting the phage that remained bound to the receptor, the eluted phage was used to infect additional host cells and the process repeated. The phages identified as binding to the receptor were retained, their DNA was isolated and sequenced, and phage clones were characterized by phage ELISA. Phage ELISA is a characterization step to confirm that the phage displayed peptide binds the receptor, and also binds to the receptor in the presence of the identified modulating compound.

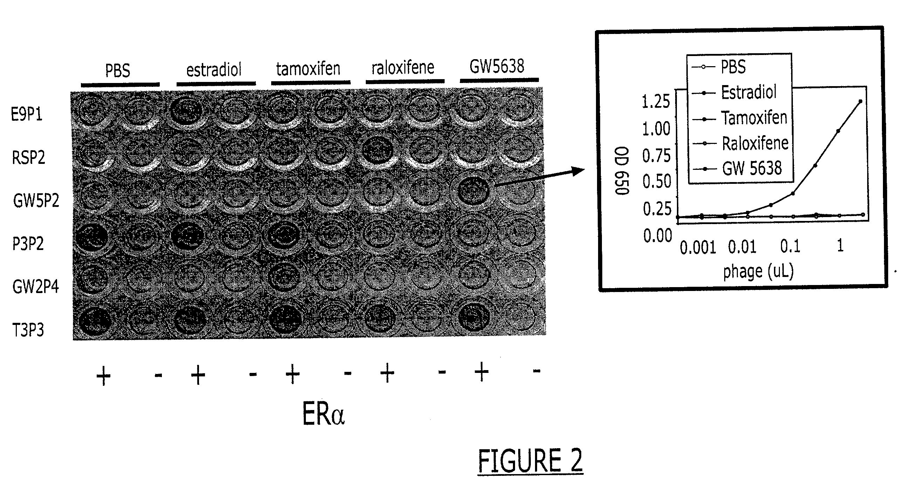

Peptides were identified that bound specifically to specified ERα/modulator pairs. As shown in **Figure 2**, selected phage clones were found that bound preferentially to estradiol + ERα (peptide E9P1) under these conditions, but did not bind to ERα + tamoxifen, ERα +raloxifene, or ERα + GW5638 under the same conditions. ERα+estradiol was shown to bind to E9P1 and P3P2 under these conditions, but not R5P2; wherease ERα+raloxifene was shown to bind to R5P2 but not E9P1 or P3P2 under these conditions.

Seven of the peptides, ranging from K7P2 (identified as a "pan agonist" selective peptide, or one that would bind to most agonist/ERα pairs to CP7P2 (identified as a "pan antagonist") were studied for their binding to a series of eight benzothiazipine ERα ligands having varied agonist - antagonist activity. **Table 1** indicates that the relative binding of the peptides to the compound/ER pairs was an indicator of relative agonist or antagonist activity of the compound. Accordingly, these peptides would be informative in Binding Profiles for ERα.

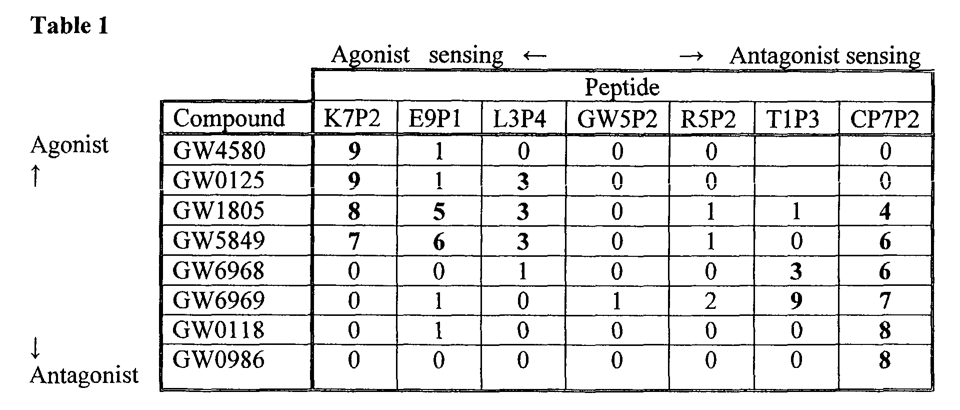

Such peptides that are known to bind preferentially to a receptor in the resence of a particular modulating compound are useful in the production of Binding Profiles and Binding Landscapes as described herein.

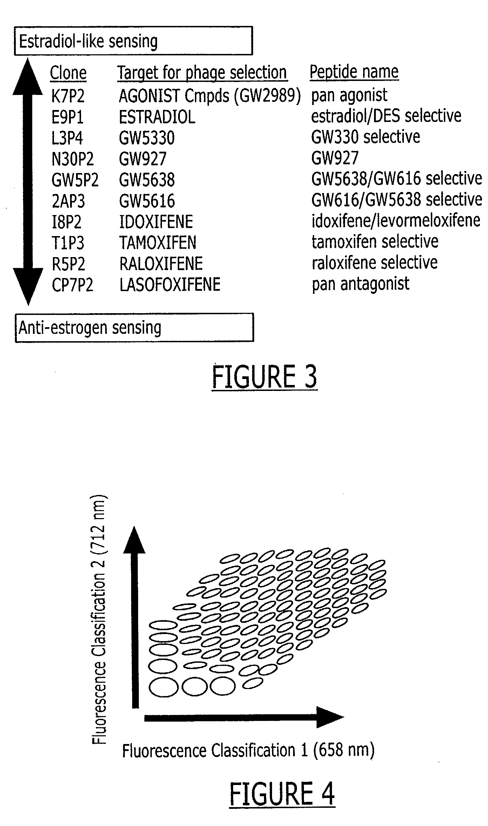

A variety of conformation-sensing peptides have been identified. **Figure 3** lists the peptide name, the conditions used for identification of the peptides, and a brief description of the specificity of the particular phage-displayed peptide.

### EXAMPLE 2

### Binding Profiles for ERα: Methods

### Example of multiplex microsphere based binding profile:

Utilizing aspects of the Multiplexing method as described in WO 01/75443, ERα Binding Profiles were generated for 145 different estrogen receptor modulator compounds, including agonists and antagonists, and those with unique tissue selective activity (e.g., idoxifene, levormeloxifene).

Forty-five binding peptides were utilized, including six peptides identified by phage display as binding to ERαLBD/modulator pairs, 32 fragments of known LXXLL-containing coactivator proteins, five fragments of LXXLL-containing coregulator proteins, and two fragments of corepressor proteins (see **Table 2**). "Fragments" were artificially synthesized based on known protein sequences; the amino acid sequence numbers corresponding to the fragments are shown in the Table.

The short sequence motif LXXLL (where L is leucine and X is any amino acid) is present in various nuclear receptor cofactor proteins, including RIP-140, SRC-1 and CBP. It has been demonstrated that these short regions, commonly known as NR boxes, are necessary and sufficient to mediate the binding of these proteins to liganded nuclear receptors. Therefor, synthetic fragments containing the motif are useful as surrogates for the complete protein in binding assays. It has been shown that the ability of SRC-1 to bind the estrogen receptor and enhance its transcriptional activity is dependent upon the integrity of the LXXLL motifs and on key hydrophobic residues in a conserved hydrophobic groove of the estrogen receptor that are required for its ligand-induced activation function. See e.g., Heery et al., Nature 387(6634):733 (1997).

**Table 2**

| | |
|---|---|
| Phage-display | K7P2 (agonist sensing) |
| identified peptides | GW5P2 (GW5638 sensing) |
| | T1P3 (tamoxifen sensing) |
| | I8P2 (levormeloxifene/idoxifene sensing) |
| | N30P2 (GW4927 sensing) |
| | R5P2 (raloxifene sensing) |
| LXXLL-containing | TRIP2 (54-80) |
| Coactivator | TRIP3 (85-109) |
| peptides | TRIP4 (23-47) |
| | TRIP8 (26-47) |
| | TRIP9 (61-85) |
| | TRIP9 (275-301) |
| | |
| | RIP140 (119-143) |
| | RIP140 (172-196) |
| | RIP140 (366-390) |
| | RIP140 (487-511) |
| | RIP140 (699-723) |
| | RIP140 (8) (805-831) |
| | RIP140 (922-946) |
| | |
| | CBP-1 (58-80) |
| | CBP-2 (346-368) |
| | CBP-3 (2055-2078) |
| | |
| | SRC-1 (1)(626-642) |
| | SRC-1 (2) (676-700) |
| | SRC-1 (3) (735-759) |
| | |
| | TIF1 (368-392) |
| | TIF1 (711-732) |
| | TIF2 (627-651) |
| | TIF2 (676-702) |
| | TIF2 (732-756) |
| | TIF2 (864-888) |
| | |
| | AIB1 (102-123) |
| | AIB1 (607-631) |
| | |
| | RAC3 (671-697) |
| | PCIP (1027-1051) |
| | |
| | P300(1)(67-93) |
| | P300(2037-2061) |
| | p300AF(176-200) |
| LXXLL-containing | DAX (1-23) |
| Coregulator | DAX (66-90) |
| peptides | DAX (132-156) |
| | |
| | SHP (7-31) |
| | SHP (104-128) |
| Corepressor | N-CoR |
| Peptides | SMRT |
| Control | - no peptide - |

The peptide sequences were synthesized with biotin at one end, and coupled to fluorescently distinct microsphere populations (5.6um streptavidin-coated latex microsphere particles). Multiple peptides of the same sequence were coupled to microspheres having the same fluorescent label, creating 46 fluorescently distinct microsphere populations (45 peptides plus no-peptide control). The microspheres populations were then multiplexed (combined). Alternatively, the ERα LBD could be coupled to the fluorescent microspheres.

The Estrogen Receptor α Ligand Binding Domain (ERα LBD) was coupled to the fluorophore Alexa 532 (Molecular Probes, Inc., Eugene, OR), which is suitable for use with 532 nm excitation sources. The excitation and emission spectra of Alexa 532 is intermediate between the green-fluorescent Alexa Fluor 488 dye and the orange-fluorescent Alexa Fluor 546 dye.

Binding Profiles using the above peptides and ERαLBD were generated for 145 compounds known to have estrogen receptor binding ability . These compounds included examples from various chemical classes, including benzothiazipines, napthalenes, triphenylethylenes, triazines, and benzopyrans. All of the compounds in this set were initially tested for binding to the ERα LBD using an estradiol displacement assay and were confirmed to have pKi ≥ 6, which is generally considered desirable in candidate pharmaceutical compound. Binding Profiles were also generated using 8 reference compounds: estradiol, 4-OH tamoxifen, raloxifene, idoxifene, levormeloxifene, GI7604, GW5616 and lasofoxifene. Cell-based assay results were known for these reference compounds in the ERE, MCF7 and Ishikawa cell-based assays (described herein).

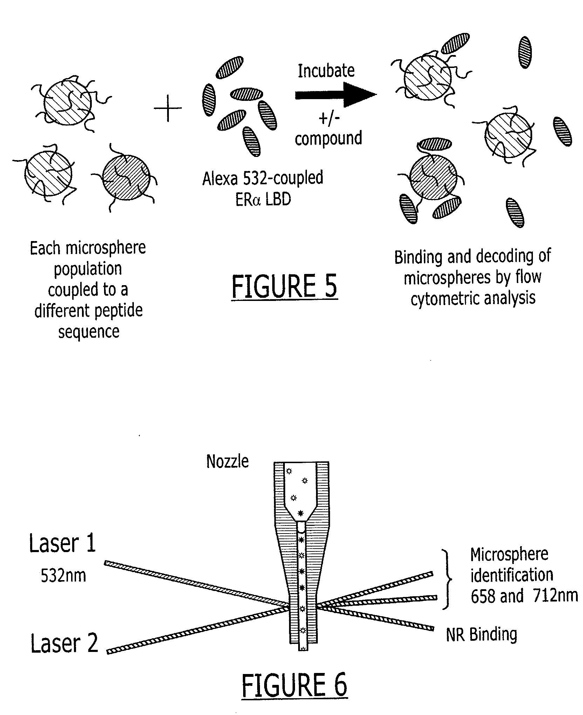

As shown in **Figures 4, 5, 6,** the multiplexed microspheres and ERα ligand binding domain are then incubated with a compound (or no compound for control), using 10nM of ERαLBD, 1uM of compound, and 2,000 microspheres of each of the 46 microsphere populations per well.

The resulting binding of peptide to LBD is detected by flow cytometric analysis as is known in the art (see, e.g. WO 01/75443). In the present example, binding of ERα to a microsphere subset was indicated by the acquisition of orange fluorescence, detected using a LX-100 instrument (Luminex Corporation, Austin, TX). The above procedure is repeated for each compound to create a library of Binding Profiles.

**Figures 7** and 8 show the extent of binding of the ERα LBD to the coactivator, coregulator, and corepressor peptides, in the presence of either no compound (basal), estradiol (1 uM) or 4-OH tamoxifen (1 uM). This data is represented in **Figure 7** as Mean Fluorescence Intensity, where fluorescence intensity indicates the amount of ERα LBD that bound to a peptide-coupled microsphere population in the presence of a particular compound. In **Figure 8**, the amount of binding is represented by subtracting basal (no compound) binding, to show the increase or decrease over basal. Figure 8 shows that for most peptides, the presence of 4-OH tamoxifen reduced the amount of peptide binding compared to basal binding, whereas estradiol increased peptide binding compared to basal levels.

Binding Profiles for each of the six phage-identified peptides described above were also generated using the same multiplexed assay as described above. The microsphere bound peptides were incubated as described above with 10nM ERαLBD and 1uM of one of four ER binding compounds: estradiol, 4-OH tamoxifen, idoxifene, levormeloxifene. Results are shown in **Figure 9**, where the binding of peptides is indicated (using basal subtraction) on the vertical axis.

As indicated by **Figure 9**, the binding of these peptides identifies whether the compound is estradiol-like (increased binding of K7P2 peptide over basal) or anti-estrogenic (4-OH tamoxifen; decreased binding of K7P2 over basal, increased binding of T1P3 over basal). The binding profiles are similar for idoxifene and levormeloxifene, two compounds known to have similar in vivo ERα activity (desirable bone protective effects).

### EXAMPLE 3: Cell-Based Assays - Methods

The present inventors utilized three ERα responsive cell-based assays in studying the effects of modulator compounds on the binding of peptides to ERα.

### a) Estrogen Response Element (ERE) assay:

The Estrogen Response Element (ERE) assay was conducted by transiently transfecting a T47D breast cell line with a plasmid containing (a) the frog vitellogenin promoter which drives expression of a secreted placental alkaline phosphatase (SPAP) reporter gene, and (b) a plasmid that expresses the full length ERα gene. The vitellogenin promoter contains the consensus vitellogenin ERE sequence (F-vitERE). See, e.g., Martinez et al., EMBO J, 6:3719(1987); Edmunds et al., Neurotoxicology 18:525 (1997). In this ERE assay, the presence of an ERα agonist increases transcriptional activity of the reporter gene.

Compounds were evaluated for their potential to either stimulate transcriptional activity (agonist mode, increasing concentrations of compound are incubated with cells in the absence of a competing compound) or inhibit transcriptional activity (antagonist mode, increasing concentrations of compound are incubated with cells in the presence of a fixed concentration of estradiol). Transcriptional activity was measured by acquisition of OD₄₀₅ which indicates SPAP activity. An EC₅₀ (agonist mode) or IC₅₀ (antagonist mode) was determined for each compound tested. A compound's ERE potency is defined as pEC50 x %max (agonist mode) or pIC50 x %min (antagonist mode).

### b) Breast cell proliferation

Cell proliferation of MCF-7 breast cancer cells was determined by [³H]-thymidine incorporation . Compounds were tested for their potential to either stimulate MCF-7 proliferation or inhibit proliferation. An EC₅₀ or IC₅₀ was determined for each compound tested. A compound's MCF-7 potency is defined as pEC50 x %CPMₘₐₓ (relative to estradiol) or pIC50 x %CPMₘᵢₙ (relative to raloxifine).

### c) Ishikawa Endometrial Cell Stimulation

The stimulation of human endometrial cells (Ishikawa cell line) was determined by increases in intrinsic alkaline phosphatase activity. Compounds were tested for their potential to stimulate endometrial cells, as compared to estradiol. The %Eₘₐₓ is defined as 100 x (spectrophotometric reading at 405nm with compound-blank)/(spectrophotometric reading at 405nm estradiol-blank).

### EXAMPLE 4: Cell Based Assays - Results

### ERE assay

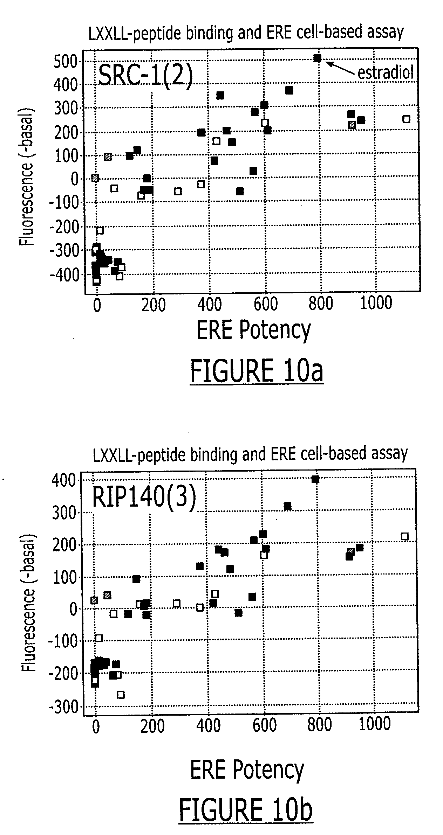

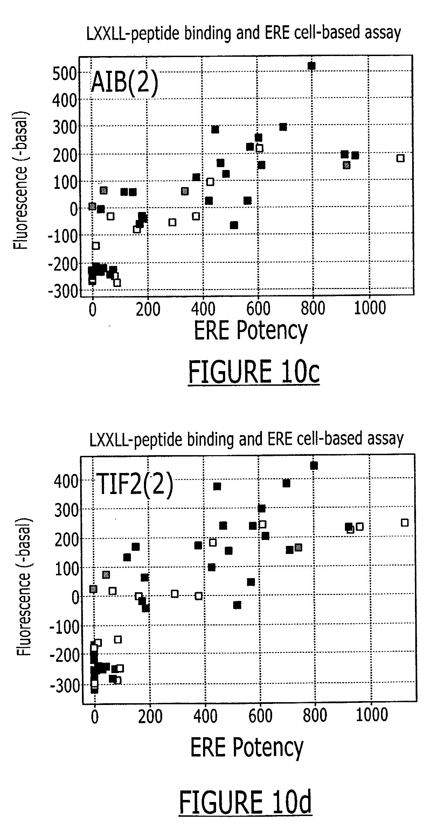

Results of the ERE cell-based assay are shown in **Figure 10**, plotted against binding assay results where multiple ER ligand compounds were tested for their effects on ERα binding to LXXLL-containing coactivator peptides. In this example, compounds were run in the agonist mode ERE assay in which compounds were tested for their ability to increase reporter gene transcription. In **Figure 10,** each square represents the results obtained with a single compound. The tool compound estradiol is included in this compound set. Each compound is shaded according to its core structure. Four example coactivator peptides are shown: SRC-1(2), AIB (2), RIP140(3) and TIF2(2). The vertical axis indicates fluorescence minus the fluorescence in the absence of compound. This difference indicates receptor binding to the respective peptide in the presence of compound. A negative value indicates the compound decreased binding of the coactivator peptide to ERα. The horizontal axis represents a measure of reporter gene expression represented as (pEC50)x%maximal response. As shown in **Figure 10a**, compounds that inhibit binding of SRC-1(2) to ERα compared to basal binding (negative value on vertical axis) also tended to group on the horizontal axis, showing they did not stimulate ERE activity (promote transcription) in the cell-based assay.

### MCF-7 Cell Proliferation

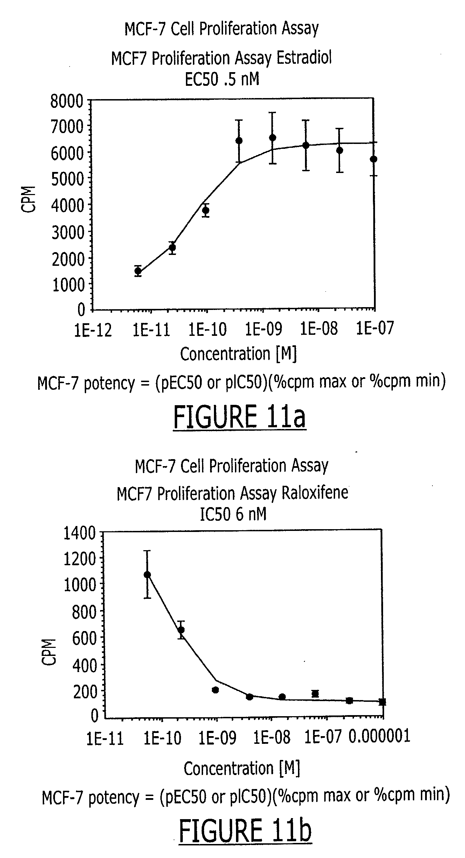

Results shown in **Figure 11** for proliferation of MCF-7 cells in the presence of estradiol or raloxifene. The vertical axis indicates counts-per-minute of ³H thymidine incorporation, as a measure of proliferation; the horizontal axis indicates concentration of the compound. These results indicate that as estradiol (agonist) concentration increased cell proliferation increased, whereas cell proliferation decreased as raloxifene (antagonist) increased.

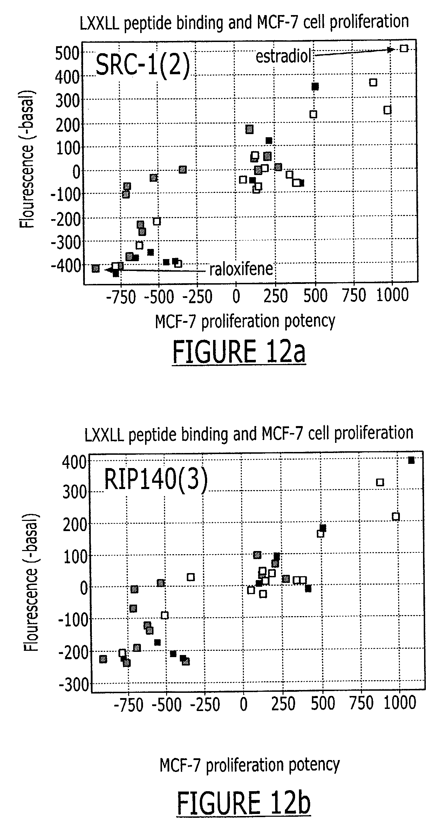

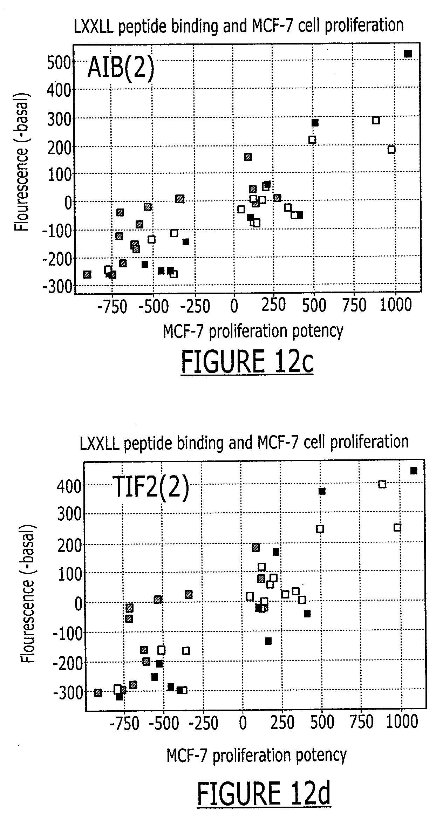

Results for the MCF-7 cell proliferation assay are shown in **Figure 12**, where multiple ER ligand compounds were tested for their effects on ERαLBD binding to LXXLL-containing coactivator proteins. In **Figure 12**, each square represents the results obtained with a single compound. The tool compounds estradiol and raloxifene were included in this set and are shown labeled. Four coactivator peptides are shown: SRC-1(2), AIB (2), RIP140(3) and TIF2(2). The vertical axis indicates fluorescence minus the fluorescence in the absence of compound. This difference indicates receptor binding to the respective receptor in the presence of compound.The horizontal axis indicates the extent of cell proliferation compared to control. A negative value indicates the compound decreased cell proliferation; a positive value indicates the compound increased cell proliferation (relative to control).

### Ishikawa Cell Stimulation Assay

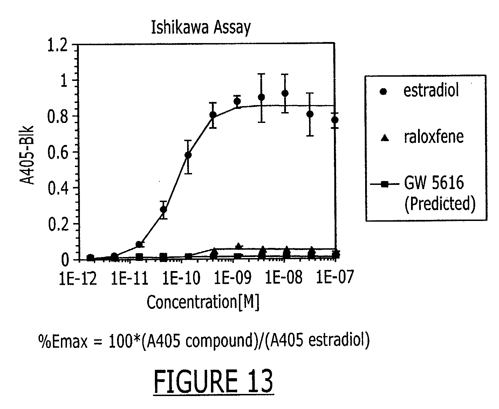

The results of the Ishikawa cell stimulation assay are shown in **Figure 13**, for estradiol and raloxifene. Estradiol increases stimulation of intrinsic alkaline phosphatase, as measured on the vertical axis. Alkaline phosphatase activity for a compound is measured by acquiring absorbance at 405nm (minus blank). As shown estradiol stimulates this activity whereas raloxifene has very low stimulatory activity.

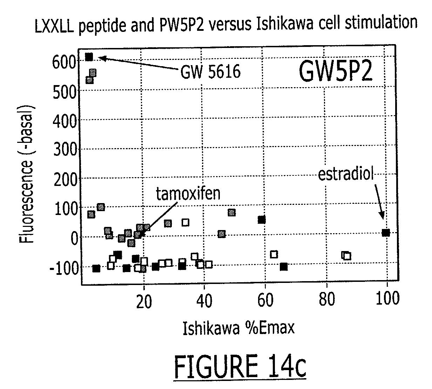

**Figure 14** shows the results of the Ishikawa cell stimulation assay for multiple ER ligand compounds, compared to their effects on ERαLBD binding to an LXXLL-containing coactivator peptide (coactivator peptide SRC-1(2) and the phage-display identified peptides T1P3 and GW5P2 ). In **Figure 14**, each square represents the results obtained with a single compound. Shown in this set of compounds are the two tool compounds estradiol and tamoxifen. The vertical axis indicates the extent of receptor binding to each of the respective peptides.. A negative value indicates the compound decreased peptide binding; a positive value indicates the compound increased peptide binding . The horizontal axis shows the amount of alkaline phosphatase activity induced by each of the compounds where the values are expressed as a percentage relative to control.

### EXAMPLE 5: Preparation of Binding Landscape

A Binding Profile provides information on the binding of a particular receptor to multiple peptides, in the presence of a particular modulating compound (or without any modulating compound, as a control) . The extent of each peptide's binding may be reported, recorded or displayed in any suitable manner as will be apparent to one skilled in the art. For example, the extent of binding may be given as an absolute value, or as a "basal subtraction" value (binding with compound minus binding without compound), or as a ratio (binding with compound/binding without compound). These values may be graphically represented as shown in the figures herein, or may be recorded and analyzed as numerical values (e.g., by storing and analyzing using a computerand appropriate software).

However, within a Binding Profile, the values provided for the peptide binding must be comparable -- i.e., obtained under similar conditions (except for the peptide variable) and reported in similar units. **Figures 7** and 8 graphically represent Binding Profiles for estradiol and 4-OH tamoxifen, and control (no compound), in bar graph form. **Figure 7** provides the data in absolute form; **Figure 8** provides the data in "basal substraction" form.

As shown in **Figures 1**6 and 17, Binding Profiles may also be represented graphically as a profile plot. **Figure 17** was prepared using Spotfire data visualization software (Spotfire Inc., Somerville Mass). Such graphic data representations assist in displaying multivariate data to human viewers.

Multiple Binding Profiles may be compiled into a Binding Landscape. **Figure 18** is a graphic representation of a Binding Landscape for three compounds (estradiol, raloxifene, and GW5616). In preparing a graphic Binding Landscape, each Binding Profile must contain data from a common set of binding peptides.

### EXAMPLE 6: Creating Binding Landscapes

The present inventors determined that Binding Profiles of reference compounds may be compiled into a Binding Landscape in order to obtain useful infromation regarding uncharacterized (test) compounds. The Binding Landscape can be utilized to predict the response of the test compound in cell-based assays, where that test compound has not previously been characterized using the cell based assays. To achieve this, a plurality of Binding Profiles for reference compounds (using the same set of binding peptides and the same receptor, but different modulating compounds) are compiled into a Binding Landscape. The Binding Landscape contains at least three, preferably about five, more preferably at least about 10, 15, 20, 25 or more reference Binding Profiles. Most preferably, the Binding Landscape contains dozens or hundreds of reference compounds, as the more reference compounds utilized the more predictive a Binding Landscape will be. Preferably, the set of binding peptides has been chosen to include peptides that are specific indicators of agonist activity, and peptides that are specific indicators of antagonist activity. Preferably the modulating compounds represented in the Binding Landscape include those having a spectrum of activity from agonist to antagonist.

As used herein, "reference compound" is not an absolute category. A compound may be uncharacterized when its Binding Profile is initially created, but as the activity of that compound is determined, it may subsequently be used as a reference compound.

Optionally, a Principal Components Analysis may be carried out on a Binding Landscape to identify those peptide variables that account for the majority of the variation seen, and to identify (and preferably omit) those peptides which do not contribute, or contribute only minimally, to the analysis. Such peptides may, for example, bind to each of the modulator/receptor combinations in a similar manner or not at all, or may replicate the binding profile of another peptide to provide cumulative information. It is most preferable to omit peptides that duplicate another peptide's responses, as including multiple peptides that respond in the same manner to the modulator compounds may bias the results. Excluding such peptide variables is termed "dimension reduction".

**Figure 21** represents a Binding Landscape for ERα LBD, which has undergone dimension reduction by Principal Component Analysis. This Binding Landscape comprises 145 different binding profiles (145 different ERα modulating compounds, originally conducted with a set of 45 binding peptides plus a no-peptide control. Principal Component Analysis using SAS JMP software indicated that 21 of the initial 46 variables accounted for >99% of the variation seen. **Figure 21** thus includes only 21 of the initial 46 peptide variables.

Once the Binding Landscape has been prepared in its final form, Peptide Profile Cluster Analysis can be conducted. The peptide Binding Profiles are grouped into clusters, based on the ratios of binding among the 21 peptides. In other words, the Binding Profiles that are most alike are classified in a hierarchical tree; similarity is based on the ratios of the data values. The ratio of the data values can be assessed visually (qualitatively) in the Binding Profiles provided herein by looking at the overall shape of the profile plot. However, it is preferred that the similarity assessment be performed quantitatively. Various quantitative methods are available to compare the data values among the Binding Profiles, as will be apparent to those skilled in the art.

The present inventors determined that the relative binding effects of a test compound *in vitro* (assessed using a Binding Landscape as set forth herein) can be used to infer the effects of the test compound in associated cell-based assays (where the cell-based assays are responsive to the same receptor tested in the Binding Profiles). Using the high-throughput Binding Landscape method, test compounds can be screened and their likely effects in cell-based assays predicted. Additionally, compounds with unusual Binding Profiles (and thus potentially novel cell-based effects) can be rapidly identified. In this manner, a series of hundreds of test compounds may be rapidly assessed, and prioritized or reduced in number for cell-based screening.

Accordingly, after the Binding Landscape is created, the compounds are clustered according to their relative effects in vitro(binding to peptides), which allows the prediction of the relative effects *in vivo* for uncharacterized compounds.

**Figure 22** shows Binding Profiles for reference compounds (those with associated cell-based assay data).

**Figure 23** shows a Binding Landscape compiled for 145 compounds, where Binding Profiles were originally conducted with 45 binding peptides plus a no-peptide control. After dimensional reduction using Principal Component Analysis, the Binding Landscape was constructed using the 21 binding peptides that provided >99% of the variation.

The individual Binding Profiles were then grouped into clusters based on binding among the 21 peptides (represented graphically by the shape of the profile). On the left of **Figure 23** is a hierarchical classification tree of Binding Profiles (a 'compound dendrogram'). The branch points of the dendrogram separate shape-based clusters. At the highest resolution, the "tree" would consist of 145 separate Binding Profiles; at the lowest resolution, the tree would consist of a single group of all 145 Binding Profiles. The most informative cut-off level in the tree lies between these two extremes, but there is no single criterium that must be used to identify an informative cut-off point. Ideally the cut-off point yields multiple clusters (as shown in **Figure 24**), where some clusters contain more than one Binding Profile.

Alternatively, in an analyzing a single uncharacterized compound, the cut-off level in the tree can be selected to provide a cluster containing that uncharacterized compound and at least one reference compound.

As shown in **Figure 24**, a cut-off point was selected that provided twelve clusters. Five of the clusters (1,2, 4, 8 and 12) contained a single Binding Profile. Three clusters contained from two to three Binding Profiles, and four clusters each contained more than ten Binding Profiles. Test compounds that fall within the same cluster as a "tool" compound are predicted to have the same in vivo effects of the tool compound. Test compounds that fall within their own cluster are predicted to have cell-based effects that differ from the 'tool' compounds.

### EXAMPLE 7

### Cell-Based Analysis and Prediction

By grouping cell-based data according to the cluster scheme identified above, it can be shown that the Binding Landscape method reveals cell-based assay trends. In the present example, three established ERα cell-based assays were utilized, as discussed in **Example 4**, above.

Of the 145 compounds represented in the Binding Profile of **Figure 23**, a subset of those had been assessed in the ERE cell-based assay. The effects of the compounds on transcriptional activity in vivo (as assessed in the ERE cell-based assay) are compared to the Clustering of Binding Profiles, to reveal associated trends. For example, as shown in **Figure 25**, in Cluster 3, the data points from the cell-based assay do not show much variation. As shown in **Figure 25**, in Cluster 3, the data points from the cell-based assay do not show much variation. Accordingly, an uncharacterized compound that fell in this cluster would be predicted to have a similar result in the ERE assay. Key associations: Increased binding of ERα LBD to coactivator peptides (including the phage-derived K7P2 peptide binding) correlated with increased ERE activity; wherease inhibition of ERα LBD binding to coactivator peptides (including K7P2) correlated with inhibition of ERE activity.

The present methods utilize whole Binding Profiles (multiple binding partners), grouped by cluster analysis, to reveal cell-based data trends specific to the Binding Profile.

**Figure 26** displays the correlation of results in the MCF-7 cell proliferation assay to the Binding Profile clusters. Again, for example, in Cluster 3, the data points for the cell-based assay do not show much variation (all are negative values). Accordingly, an uncharacterized modulating compound with a Binding Profile in this cluster can be predicted to have similar results when tested in the MCF-7 cell-based assay. Key associations: ERα LBD binding to coactivator peptides (including K7P2 peptide binding) correlated with increased MCF-7 cell proliferation.

In **Figure 27**, the results for the Ishikawa cell-based assay are grouped tightly in Cluster 3 as an example; accordingly, an uncharacterized modulating compound with a Binding Profile in this cluster is expected to have similar results when tested in an Ishikawa cell-based assay. Key associations: Enhanced binding of ERα LBD to the peptides GW5P2 and T1P3 correlates with low Ishikawa stimulation and increased binding of ERα LBD to coactivator peptides (including K7P2) correlates with high Ishikawa stimulation.

### EXAMPLE 8: Further refinement of Binding Landscape

In some cases a Binding Landscape cluster will contain similar binding profiles, yet the results of an associated cell-based assay are not similar (i.e., the Binding Landscape cluster is not predictive for results in that cell-based assay). In such instances that cluster may be further resolved by the addition of additional binding peptide data to the Binding Profiles. This may be carried out by screening additional phage-displayed peptides (as discussed in **Example 1, above**) to identify additional appropriate binding peptides, or by screening additional known coactivator, corepressor, or coregulator proteins, or to screen DNA fragments that represent promoter elements to identify those that show variation in binding among the receptor/modulator combinations in the unresolved cluster.

### EXAMPLE 9: Identification of compounds with unique Binding Profiles

Where a Binding Landscape has been prepared using known receptor ligands and uncharacterized modulating compounds, and has been resolved into clusters using a middle point on the dendrogram, a cluster containing a single uncharacterized compound indicates that the uncharacterized compound has unique properties compared to the known receptor ligands.

## Claims

1. A method of predicting the effect of a pharmaceutical compound in a cell-based assay that is responsive to modulation of a nuclear receptor, comprising:
a) selecting a plurality of reference compounds whose effects in a cell-based assay are known, where said cell-based assay is responsive to modulation of a nuclear receptor;
b) selecting a plurality of binding partners known to bind to said nuclear receptor;
c) contacting said binding partners and said nuclear receptor in the presence of a reference compound, under conditions that allow binding, and detecting the extent of binding achieved, such that the compound's binding profile can be **characterized by** the relative differences between the binding results for that compound ;
d) repeating step (c) for each of said reference compounds;
e) detecting, for at least one test compound whose effects in said cell-based assay are not known, the extent of binding of said nuclear receptor to each of said binding partners in the presence of said test compound, such that the test compound's binding profile can be **characterized by** the relative differences between the binding results for that compound; and
f) comparing the binding profile of each reference compound to the binding profile of the test compound, to identify the reference compound having the binding profile that is most similar to the test compound binding profile;
where identification of a reference compound binding profile that is similar to the test compound binding profile indicates that the test compound will achieve results in said cell-based assays that are similar to the results achieved by the reference compound.

2. A method according to claim 1 where said binding partners are proteins, peptides, or DNA sequences.

3. A method according to claim 1 where said binding partners are selected from corepressor proteins, coactivator proteins, coregulator proteins, and fragments thereof that contain a binding domain for said nuclear receptor.

4. A method according to claim 1 where comparing step (f) is carried out by comparing the euclidean distance between the slopes of the reference and test compounds' binding results for each binding partner.

5. A method according to claim 1 where said nuclear receptor is Estrogen Receptor alpha (ERα).

6. A method according to claim 5 where said reference compounds include raloxifene and estradiol.

7. A method according to claim 5 where said cell-based assay is selected from ERE assay, MCF-7 cell proliferation assay, and Ishikawa cell stimulation assay.

8. A method of predicting the effect of a pharmaceutical compound in a cell-based assay that is responsive to modulation of a GPCR, comprising:
a. selecting a plurality of reference compounds whose effects in a cell-based assay are known, said cell-based assay responsive to modulation of a G-protein coupled receptor (GPCR);
b. selecting a plurality of binding partners known to bind to said GPCR;
c. contacting said binding partners and said GPCR in the presence of a reference compound, under conditions that allow binding, and detecting the extent of binding achieved, such that the compound's binding profile can be **characterized by** the relative differences between the binding results for that compound ;
d. repeating step (c) for each of said reference compounds;
e. detecting, for at least one test compound whose effects in said cell-based assay are not known, the extent of binding of said GPCR to each of said binding partners in the presence of said test compound, such that the test compound's binding profile can be **characterized by** the relative differences between the binding results for that compound; and
f. comparing the binding profile of each reference compound to the binding profile of the test compound, to identify the reference compound having the binding profile that is most similar to the test compound binding profile;
where identification of a reference compound binding profile that is similar to the test compound binding profile indicates that the test compound will achieve results in said cell-based assays that are similar to the results achieved by the reference compound.

9. A method according to claim 1 where said binding partners are proteins or peptides.

10. A method according to claim 1 where comparing step (f) is carried out by comparing the euclidean distance between the slopes of the reference and test compounds' binding results for each binding partner.

11. A method of predicting the effect of a pharmaceutical compound in a cell-based assay that is responsive to modulation of a 7TM receptor, comprising:
a. selecting a plurality of reference compounds whose effects in a cell-based assay are known, said cell-based assay responsive to modulation of a 7-transmembrane receptor (7TM receptor);
b. selecting a plurality of binding partners known to bind to said 7TM receptor;
c. contacting said binding partners and said 7TM in the presence of a reference compound, under conditions that allow binding, and detecting the extent of binding achieved, such that the compound's binding profile can be **characterized by** the relative differences between the binding results for that compound ; repeating step (c) for each of said reference compounds;
d. for at least one test compound whose effects in said cell-based assay are not known, detecting the extent of binding of said 7TM receptor to each of said binding partners in the presence of said test compound, such that the test compound's binding profile can be **characterized by** the relative differences between the binding results for that compound;
e. detecting, for at least one test compound whose effects in said cell-based assay are not known, the extent of binding of said 7TM to each of said binding partners in the presence of said test compound, such that the test compound's binding profile can be **characterized by** the relative differences between the binding results for that compound; and
f. comparing the binding profile of each reference compound to the binding profile of the test compound, to identify the reference compound having the binding profile that is most similar to the test compound binding profile
where identification of a reference compound binding profile that is similar to the test compound binding profile indicates that the test compound will achieve results in said cell-based assays that are similar to the results achieved by the reference compound.

12. A method according to claim 1 where said binding partners are proteins or peptides.

13. A method according to claim 1 where comparing step (f) is carried out by comparing the euclidean distance between the slopes of the reference and test compounds' binding results for each binding partner.

14. A method of predicting the effect of a pharmaceutical compound in a cell-based assay that is responsive to modulation of an ion channel, comprising:
a. selecting a plurality of reference compounds whose effects in a cell-based assay are known, said cell-based assay responsive to modulation of an ion channel;
b. selecting a plurality of binding partners known to bind to said ion channel;
c. contacting said binding partners and said ion channel in the presence of a reference compound, under conditions that allow binding, and detecting the extent of binding achieved, such that the compound's binding profile can be **characterized by** the relative differences between the binding results for that compound ; repeating step (c) for each of said reference compounds;
d. repeating step (c) for each of said reference compounds;
e. detecting, for at least one test compound whose effects in said cell-based assay are not known, the extent of binding of said ion channel to each of said binding partners in the presence of said test compound, such that the test compound's binding profile can be **characterized by** the relative differences between the binding results for that compound; and
f. comparing the binding profile of each reference compound to the binding profile of the test compound, to identify the reference compound having the binding profile that is most similar to the test compound binding profile;
where identification of a reference compound binding profile that is similar to the test compound binding profile indicates that the test compound will achieve results in said cell-based assays that are similar to the results achieved by the reference compound.

15. A method according to claim 1 where said binding partners are proteins or peptides.

16. A method according to claim 1 where comparing step (f) is carried out by comparing the euclidean distance between the slopes of the reference and test compounds' binding results for each binding partner.
